# EUROPEAN PATENT SPECIFICATION

(11) **EP 4 384 059 B1**
(45) Date of publication and mention of the grant of the patent: **19.03.2025**
(21) Application number: 22802719.9
(22) Date of filing: 02.11.2022
(51) Int. Cl.: A61B 1/00, A61B 1/005

(54) **ENDOSCOPE WITH INSERTION TUBE HAVING ADJACENT CUTS WITH UNEQUAL SPACING AND METHOD OF MANUFACTURING SUCH AN ENDOSCOPE**
ENDOSKOP MIT EINFÜHRROHR MIT BENACHBARTEN EINSCHNITTEN MIT UNGLEICHEM ABSTAND UND VERFAHREN ZUR HERSTELLUNG SOLCH EINES ENDOSKOPS
ENDOSCOPE DOTÉ D'UN TUBE D'INSERTION AYANT DES DÉCOUPES ADJACENTES AVEC ESPACEMENT INÉGAL ET PROCÉDÉ DE FABRICATION D'UN TEL ENDOSCOPE

(30) Priority: 02.11.2021 DE 102021128447
(43) Date of publication of application: 19.06.2024
(73) Proprietor: HOYA CORPORATION, Shinjuku-ku Tokyo 160-8347 (JP)
(72) Inventor: DO, Anh Minh, 86316 Friedberg (DE)
(74) Representative: Lucke, Andreas
(86) International application number: PCT/IB2022/060548
(87) International publication number: WO 2023/079451

(56) References cited:
- EP-A2- 2 742 879
- WO-A1-2020/089684
- US-A1- 2016 287 054
- US-A1- 2017 079 505

## Description

The present disclosure relates to an endoscope with an insertion tube having adjacent cuts with unequal spacing and to a method of manufacturing such an endoscope.

An endoscope is a device that allows for examining the inside of living organisms, but also technical cavities. An important part of an endoscope is the flexible insertion tube. The requirements for an insertion tube are high and diverse. On the one hand, it must be flexible so that it can be inserted into the human body. On the other hand, the insertion tube must have a certain stiffness. During the examination, the physician must be able to push and turn the insertion tube using the control body. For this, the insertion tube must be stiff (rigid) enough not to be kinked or twisted. Conventional insertion tubes therefore require a very complex design and high manufacturing costs in order to meet these requirements.

In order to meet all requirements, the insertion tube must have various properties. Three of the most important properties of an insertion tube are bending flexibility, torsional rigidity (torsional resistance) and dimensional stability (form/shape stability). On the one hand, it must be bendable in order to be inserted into the (e.g. human) body to be examined. On the other hand, the insertion tube must have a high torsional rigidity in order to be able to transmit the torque generated by the user by rotation of a control body on to the distal end. Furthermore, the insertion tube must not deform when it is bent or twisted.

The requirement that an insertion tube must possess the abovementioned properties at the same time is in itself a technical contradiction. An element is normally stiff and dimensionally stable if it has high torsional rigidity. However, if the element has high bending flexibility, then it does not have high torsional rigidity and is not dimensionally stable.

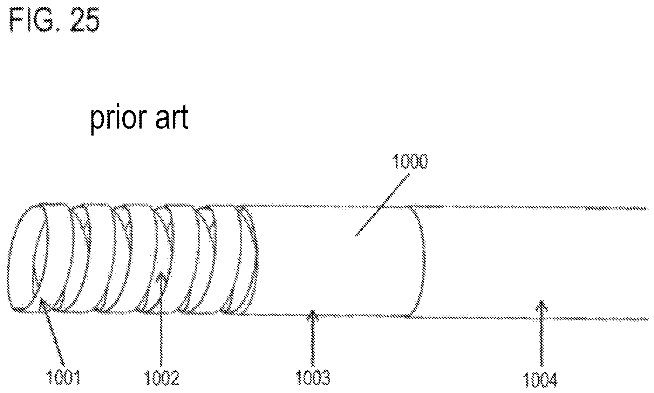

To meet the above requirement, developers have been trying for some time to construct the base portion of the insertion tube from multiple components. A known design of a base portion of the insertion tube can be seen in Fig. 25.

In the known solution of Fig. 25, three different components are assembled to achieve the relevant properties of the base portion of an insertion tube 1000, namely high flexibility, high torsional rigidity and high dimensional stability.

A plastic coating 1004 is heated until the material on its inner side partially melts and enters into gaps in a metal mesh 1003. This combination provides the base portion of an insertion tube 1000 with high torsional rigidity and high bending flexibility. However, dimensional stability is still lacking here. For this, two metal sheet spirals 1001 and 1002 arranged in opposite directions are used. These metal sheet spirals 1001 and 1002 ensure that the insertion tube is dimensionally stable. The combination described now provides the insertion tube 1000 with the three necessary properties mentioned: namely, high flexibility, high torsional rigidity and high dimensional stability.

One disadvantage of this complex design is in economic terms. Three components are assembled together in a complex manufacturing process. Both the materials and the manufacturing process cause high manufacturing costs.

US 2016/287054 A1 discloses an endoscope that includes a long insertion portion sequentially provided with first and second bending portions and a flexible tube portion, from a distal end side; first and second tubular portions including first and second notch portions provided inside of the first and second bending portions, respectively; and a third tubular portion including a third notch portion provided inside of the flexible tube portion, wherein the first to third tubular portions are formed in one tubular member, the first notch portion is formed by first notches bendable in at least two directions, the second tubular portion includes third notches formed in a direction different from the two directions of second notches.

WO 2020/089684 A1 discloses a method for producing an insertion tub of an endoscope from a tube element, wherein the insertion tube has a proximal, passive, flexible section and a distal, anglable section, wherein cuts are made in the proximal, passive, flexible section in order to permit a bending of the proximal, passive, flexible section. In this method, the cuts in the proximal, passive, flexible section are designed such that neighbouring cuts are unevenly spaced apart. The disclosure also relates to an endoscope having an insertion tube of this type.

EP 2 742 879 A2 discloses an instrument that has a cylindrical element which is insertable through a catheter into a body cavity. The cylindrical element has a distal end having a radially expandable functional portion and a stable diameter, and a cylindrical guide portion, which is arranged proximal to the functional portion. The distal end is structured for the formation of the functional portion such that the functional portion includes a web forming an integral extension of the guide portion. The element is formed by a solid wire or a hollow wire and formed from a composite material with two components.

It is an object of the present disclosure to provide a method of manufacturing an insertion tube of an endoscope and an endoscope with an insertion tube, which are less complex and by which costs can be reduced.

This object is met by an endoscope with the features of claim 1. A corresponding method is provided in claim 7. Further examples thereof are detailed in the dependent claims.

The disclosure is directed to an endoscope having an insertion tube, wherein the insertion tube has a proximal passive flexible section and a distal bending section, cuts are provided in the proximal passive flexible section to allow for bending the proximal passive flexible section, adjacent cuts in the proximal passive flexible section are unequally spaced, the proximal passive flexible section has secondary cuts adjacent to main cuts, the secondary cuts being arranged closer in a longitudinal direction of the proximal passive flexible section to the adjacent main cuts on one side of the secondary cuts than to the adjacent main cuts on the other side of the secondary cuts, and the main cuts extend along the circumference of the proximal passive flexible section in an interrupted manner such that uncut bridges (stays) remain between main cut portions lying on a circumferential line, wherein the proximal passive flexible section has the main cuts, wherein at least within a subzone in the proximal passive flexible section the main cuts are unequally spaced from each other (unequally spaced apart from each other) in the longitudinal direction of the proximal passive flexible section.

In the insertion tube of the endoscope according to the disclosure, cuts are formed with unequal spacing. The spacing of cuts formed in the insertion tube is thus different from each other. The cuts may be formed perpendicular to the axis of the insertion tube. In the proximal passive flexible section, main cuts are provided. At least within a subzone in the proximal passive flexible section, the main cuts are unequally spaced from each other in the longitudinal direction of the proximal passive flexible section.

This endoscope offers high flexibility in the shaping of bending angles in the proximal passive flexible section.

The main cuts may be spaced from each other (spaced apart from each other) in the longitudinal direction of the proximal passive flexible section with continuously increasing spacing. The main cuts may be spaced from each other in a distal direction of the proximal passive flexible section with continuously increasing spacing. Thus, the potential bending angle of the proximal passive flexible section decreases in the distal direction of the proximal passive flexible section and the bending (angulation, deflection) increases accordingly.

The main cuts may be spaced from each other in the longitudinal direction of the proximal passive flexible section with continuously decreasing spacing. The main cuts may be spaced from each other in the distal direction of the proximal passive flexible section with continuously decreasing spacing. Thus, the potential bending angle of the proximal passive flexible section increases in the distal direction of the proximal passive flexible section and the bending (angulation, deflection) decreases accordingly.

At least within a first subzone in the proximal passive flexible section, the main cuts are spaced from each other in the longitudinal direction of the proximal passive flexible section with continuously increasing spacing, and at least within a second subzone in the proximal passive flexible section, the main cuts are spaced from each other in the longitudinal direction of the proximal passive flexible section with continuously decreasing spacing. A proximal passive flexible section may be implemented in which a decreasing potential bending angle of the first subzone is combined with an increasing potential bending angle of the second subzone.

The first subzone and the second subzone may border (abut) on each other. A proximal passive flexible section may be implemented in which a decreasing potential bending angle of the first subzone and an increasing potential bending angle of the second subzone are combined directly/back-to-back. The change of the potential bending angle can be realized within a short longitudinal extent.

Between the first subzone and the second subzone, a third subzone may be arranged in which the main cuts are equally spaced from each other in the longitudinal direction of the proximal passive flexible section. The change of the potential bending angle may be realized via a deliberate smooth transition of a constant potential bending angle (in the third subzone).

The aspects of the present disclosure explained above may be suitably combined.

### BRIEF DESCRIPTION OF THE DRAWINGS

Fig. 1 shows a schematic side view of an endoscope in which the disclosure can be employed.
Fig. 2 shows a partial schematic view of an insertion tube according to the disclosure.
Fig. 3 shows a partial schematic side view of a part of a proximal passive flexible section of the insertion tube of a first example according to the disclosure.
Fig. 4 shows a partial perspective view of the part of the proximal passive flexible section of Fig. 3.
Fig. 5 shows a detail of the proximal passive flexible section of Fig. 3 for explaining the bending rigidity.
Fig. 6 shows a relationship between deformation and spacing between pipe cuts during bending with regard to bending rigidity.
Fig. 7 shows a detail of the proximal passive flexible section of Fig. 3 for further explanation of the bending rigidity.
Fig. 8 shows a relationship between deformation and spacing between pipe cuts during bending with regard to torsional rigidity.
Fig. 9 shows a detail of the proximal passive flexible section of Fig. 3 for explaining the torsional rigidity.
Fig. 10 shows a partial perspective view of the part of the proximal passive flexible section of the first example of Fig. 3 under torsional stress.
Fig. 11 shows a partial schematic view of a transition portion between the distal bending section and the proximal passive flexible section of the insertion tube according to the disclosure, wherein a guide spring fixing section is shown.
Fig. 12 shows a partial perspective view of the guide spring fixing section of Fig. 11 from another side.
Fig. 13 shows a partial schematic view of a part of the bending section of the insertion tube according to the disclosure.
Fig. 14 shows a partial schematic view of the part of the bending section of the insertion tube according to the disclosure, showing a view from the direction of an arrow I in Fig. 13.
Fig. 15 shows a partial schematic view of a part of the bending section of the insertion tube according to the disclosure, wherein a cable guide is shown.
Fig. 16 shows a partial perspective view of the cable guide of Fig. 14.
Fig. 17 shows a partial schematic side view of the bending section of the insertion tube according to the disclosure.
Fig. 18 shows a partial schematic plan view of the bending section of Fig. 17.
Figs. 19 to 21 each show a partial perspective view of the distal end of the bending section.
Fig. 22 shows a partial perspective view of the pull cable anchoring at the distal end of the bending section.
Fig. 23 shows a view corresponding to Fig. 22 from another side.
Fig. 24 shows a partial schematic view of the proximal passive flexible section in a second example.
Fig. 25 shows a partial perspective view of an insertion tube from the prior art.
Fig. 26 shows a partial schematic view of the proximal passive flexible section in a third example in two variants in comparison with the previous examples.
Fig. 27 shows a partial schematic view of the proximal passive flexible section in the third example in further variants.

### DESCRIPTION OF THE EXAMPLES

Hereinafter, the present disclosure is described in detail with reference to the drawings by means of examples.

### First example

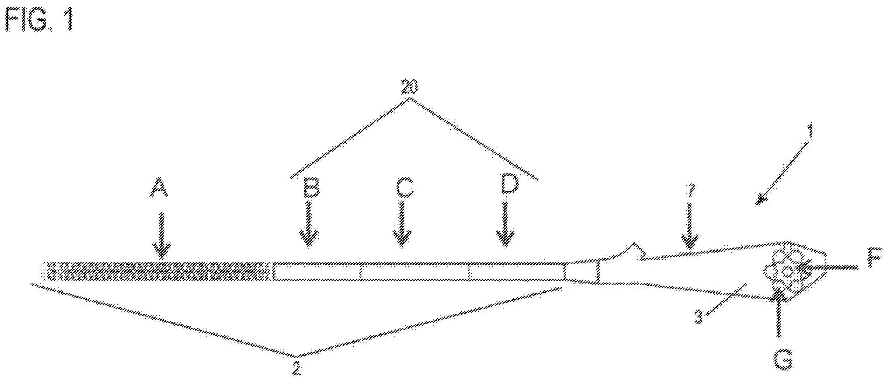

Referring now to Figures 1 to 23, a first example of the present disclosure is described below.

First, Fig. 1 shows a schematic side view of an endoscope 1 in which the disclosure can be employed. As can be seen from Fig. 1, such an endoscope 1 has an insertion tube 2 arranged on the distal side of a control body 3. The control body 3 serves as the operating unit of the endoscope 1. The control body 3 includes a handle unit 7.

The insertion tube 2 is a cylindrical pipe-like or tube-like structure.

The insertion tube 2 is described in more detail below in the direction in which it is inserted in a patient. The insertion tube 2 is inserted with the distal end first/in front.

At the distal side, the insertion tube 2 has a distal bending section (angulation section, deflecting section) A. The bending section A can be bent laterally relative to the proximal part of the insertion tube 2 by means of one or more control wires (cable pull or cable pulls). The control wire (steering wire) or cable pull, hereinafter referred to only as control wire, is supported in the interior of the insertion tube 2 guided in an direction of extension of the insertion tube 2 at an inner circumferential surface of the insertion tube 2.

The distal end of the control wire is anchored at the distal side of the bending section A. The proximal end of the control wire is connected to a control element (steering element) arranged in the control body 3. This control element tensions the control wire to bring about a desired bending of the bending section A.

Proximally from the bending section A, the insertion tube 2 is configured as a flexible tube member forming a proximal passive flexible section 20. When the insertion tube 2 is inserted, the flexible section 20 follows the bending section A.

In Fig. 1, it is indicated that the flexible section 20 is configured along its longitudinal direction into zones of varying flexibility. For example, as viewed in the proximal direction, the flexible section 20 has a first zone B, a second zone C, and a third zone D. The first zone B forms a distal portion (distal region), the second zone C forms a middle portion (middle region, central portion), and the third zone D forms a proximal portion (proximal region).

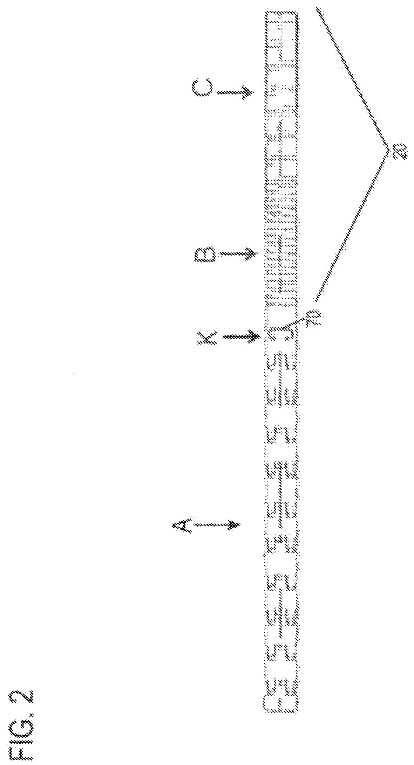

The third zone D is not shown in the partial view of Fig. 2.

To avoid kink bending between the bending section A and the first zone B, the first zone B is preferably provided with the highest flexibility among the zones of the flexible section 20. Since the first zone B is provided with a very high degree of flexibility, there is no abrupt transition of the flexibility between the bending section A and the first zone B.

The second zone C has a lower flexibility than the first zone B. The third zone D in turn has a lower flexibility than the second zone C.

The insertion tube 2 according to the disclosure is formed of one piece. That is, at the transition from the bending section A to the flexible section 20, there are not two elements that are joined together. Thus, the distal bending section A and the proximal passive flexible section 20 with the three zones B, C and D are formed of a single pipe or tube.

On the proximal side, the insertion tube 2 is fixed at the distal end of the control body 3. The insertion tube 2 can be fixed at the control body 3, for example, by a locking/fixing ring, a sealing ring or directly. The insertion tube 2 may for example be glued or screwed to the control body 3. The control body 3 has a first control wheel (steering wheel) F as a first control element for controlling a control wire or cable pull, and a second control wheel G as a second control element for controlling a control wire or cable pull. The first control wheel F may, by pulling a control wire or cable pull, bend (angle, deflect) the bending section A in a first plane (e.g., towards and away from the viewer in Fig. 1). The second control wheel G can, by pulling a control wire or cable pull, bend (angle, deflect) the bending section A in a second plane that is perpendicular to the first plane (e.g., up and down in Fig. 1).

The bending section A can be bent, for example, by 200 - 270 degrees. This is sufficient for most applications. In a special form, the bending section A can even be bent by 300 degrees.

The insertion tube 2 according to the disclosure and its manufacture are described in more detail below.

The entire insertion tube 2 is formed of/from a single pipe element (pipe member) or tube element (tube member, tubular piece/element), hereinafter referred to simply as pipe element. The pipe element is a pipe (or tube) of preferably relatively hard material. A pipe made of stainless steel is particularly preferred. However, a pipe made of hard plastic can also be used. In principle, however, any material suitable for medical purposes can be used.

Cuts are provided in the pipe element by a laser cutting machine, as explained in more detail below. After providing the cuts, certain parts of the pipe element are bent, as explained in more detail below. The manufacture of the base body (main body) of the entire insertion tube 2 does not require any further process steps other than the providing of cuts and the bending. Thereafter, the base body of the insertion tube 2 may be provided with a control wire and surrounded (sheathed) with a cover element (sheath element).

The individual sections of the insertion tube 2 are described in more detail below.

### Flexible section 20

The flexible section 20 forms the proximal part of the insertion tube 2 according to the disclosure. The flexible section 20 has the three zones B, C and D, each with a different flexibility.

Fig. 1 shows the proximal passive flexible section 20 for improved clarity as if the three zones B, C and D were of equal length to each other along the longitudinal direction of the insertion tube 2. This is, of course, not the case. The middle zone C is longer than the transition portion B and the connecting portion D. Of the three zones B, C and D, the middle zone C is the longest in the proximal passive flexible section 20. In other words, the actual proximal passive flexible section 20 is formed by the structure of the middle zone C. The bending properties, elasticity and torsional rigidity of the proximal passive flexible section 20 are implemented by the structure of the middle portion C.

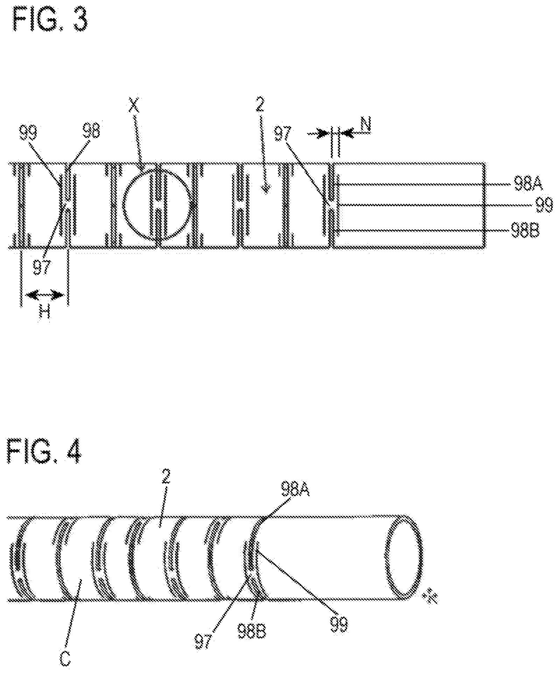

Hereinafter, the structure of the middle portion C and thus of the actual proximal passive flexible section 20 is described in more detail with reference to Figures 3 - 10.

Fig. 3 shows a partial schematic side view of a part of a proximal passive flexible section of the insertion tube of a first example according to the disclosure.

Fig. 4 shows a partial perspective view of the part of the proximal passive flexible section of Fig. 3.

The cut structure of the first example according to the disclosure can be seen from Figures 3 and 4.

In the manufacture of this cut structure, a pipe (or tube) 2 is used as raw material. The pipe 2 has an axis and a longitudinal extent. The pipe 2 is made of a sufficiently hard material. For example, stainless steel may be used. Plastic or a nickel-titanium alloy such as nitinol may also be used. The pipe 2 later forms the insertion tube according to the disclosure.

The pipe 2 has a shape (or form) that initially is not flexible. The pipe 2 has a high torsional rigidity and a high dimensional stability.

In this pipe 2, main cuts 98 are formed, preferably by laser, on the circumference in circumferential direction at predetermined spacings (distances, intervals) H. The circumferential direction refers to a direction that runs perpendicular to the axis of the pipe 2. Along the pipe 2, the spacing H is the same in each case.

The main cuts 98 penetrate the thickness of the wall of the pipe 2. The main cuts 98 extend in the circumferential direction of the pipe 2 over almost half a circumferential length (circumference). Thus, two circumferentially consecutive main cut portions 98A, 98B are formed per circumferential line. Between the respective main cut portions 98A, 98B there is a bridge (stay) 97 at which the material of the pipe 2 is not cut. Viewed in the longitudinal direction of the pipe 2, the portions in front of and behind (proximal and distal to) the respective main cut 98 are connected to each other via the bridge 97. Thus, at each circumferential line for the main cut 98 there are two bridges 97. At each circumferential line for the main cut 98, the two bridges 97 are arranged diametrically opposed. Viewed in the circumferential direction, a length of a main cut portion 98A, 98B plus a length of the bridge 97 corresponds to exactly 180°. The lengths of the main cut portion 98A and the main cut portion 98B are equal to each other.

From main cut 98 to main cut 98, along the longitudinal direction of the pipe 2, the bridges are offset by 90° with respect to each other, as can be seen from Figures 3 and 4.

Secondary cuts (auxiliary cuts, side cuts) 99 are formed proximally and distally of each bridge 97 in the longitudinal direction of the pipe 2. The secondary cuts 99 run parallel to the main cut portions 98A, 98B. The length of the secondary cuts 99 in the circumferential direction is longer than the length of the bridge 97 in the circumferential direction. The lengths of the secondary cuts 99 are equal to each other.

In the longitudinal direction of the pipe 2, the spacing (distance) N of each secondary cut 99 from its adjacent main cut portions 98A, 98B is smaller than the spacing (distance) H of the main cuts 98. Thus, a proximal secondary cut 99 and a distal secondary cut 99 are associated with each main cut 98 including the two main cut portions 98A, 98B.

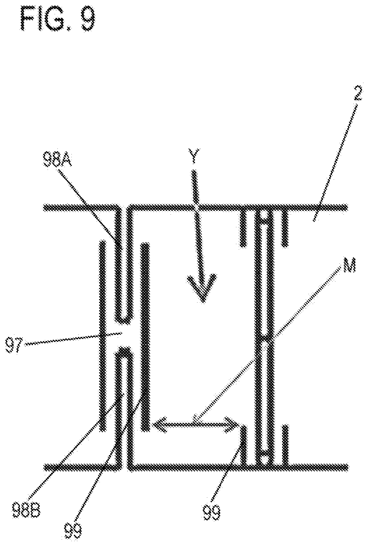

In the longitudinal direction of the pipe 2, the spacing N of each secondary cut 99 from its adjacent main cut portions 98A, 98B is also smaller than the spacing (distance) M of each secondary cut 99 from its adjacent secondary cut 99 associated with the next main cut 98, see Fig. 9.

The main cuts 98 and secondary cuts 99 change the characteristic of the pipe 2. The pipe 2 becomes flexible. The flexibility and other properties/characteristics of the pipe 2 strongly depend on, among other things, the structure of the cuts 98, 99. More specifically, the cut width, cut length and spacings of the pipe cuts (tube cuts), among others (in addition to the material), are important factors affecting the properties of the pipe 2.

In the portion X (region X) there is the cut structure which is responsible for the emergence of the high flexibility of the pipe 2.

The relationship between the deformation and the spacing between pipe cuts during bending is explained below.

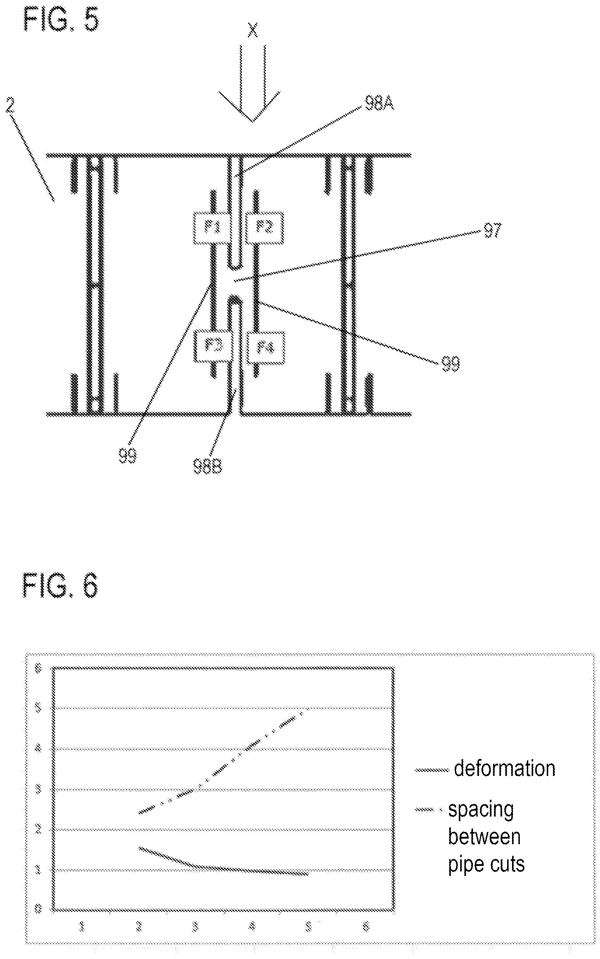

A pipe (or tube) in its original form without cuts has a certain bending rigidity (bending resistance). As soon as this pipe is cut, the bending rigidity decreases according to the shape and number of cuts provided in the pipe. The graphical representation in Fig. 6 shows the relationship between the deformation and the spacing between pipe cuts when the pipe is bent.

Fig. 6 shows the results of a bending simulation of a pipe provided with cuts. The deformation of a pipe with cuts during a bending process is shown.

The dashed-dotted line with two points indicates the spacing of a cut to its adjacent cut.

The solid line indicates the deformation of the pipe during bending.

The ordinate and the abscissa each show length unit values (e.g. mm).

The following can be seen from Fig. 6: the greater the spacing between the pipe cuts, the greater the bending rigidity (the lower the deformation). If the spacing between the pipe cuts becomes infinite, the pipe 2 reaches its original highest bending rigidity.

Since an insertion tube of an endoscope requires a low bending rigidity (and thus a high flexibility), a spacing between the pipe cuts must consequently be as small as possible.

According to the disclosure, the structure in the portion X is configured such that the cuts 98 and 99 are close together (small spacing N) and four spring-like segments F1, F2, F3 and F4 are formed. If the cut pipe 2 is now bent, the segments F1, F2, F3 and F4 are pulled apart and thus a spring-like counterforce is generated. When the pipe 2 is relieved of load after bending, the counterforce acts on the pipe 2 such that it regains its straight shape. Along the longitudinal direction of the pipe 2, this structure of the portion X is arranged repeatedly offset by 90° along the entire length of the proximal passive flexible section C of the pipe 2. As a result, the pipe 2 is uniformly flexible in all directions.

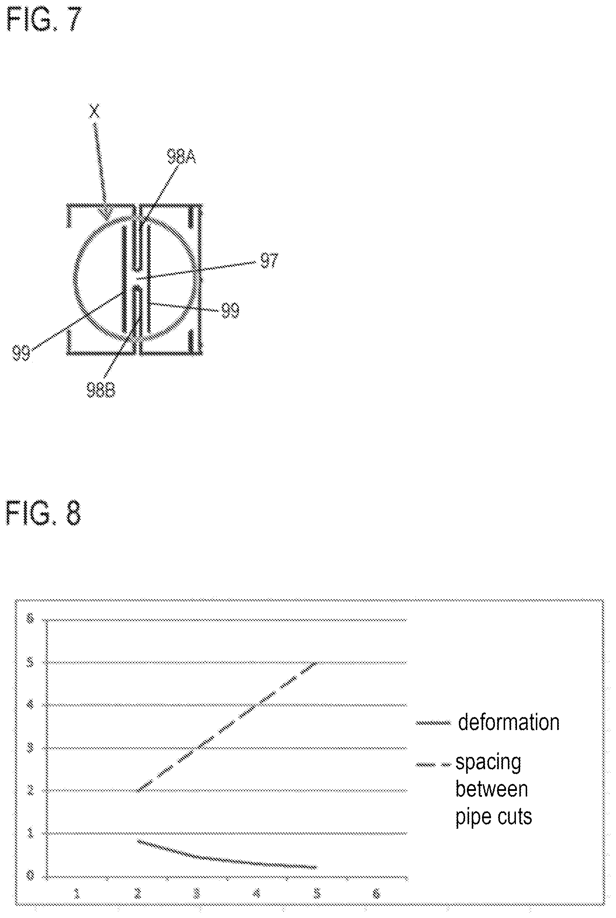

Fig. 7 shows the portion X as an enlarged section. In the structure of a main cut 98, made up of a first main cut portion 98A and a second main cut portion 98B, with the associated secondary cuts 99 in the portion X, the spacing N between the main cut portions 98A, 98B and the associated secondary cuts 99 should be as small as possible to provide a high degree of flexibility.

The torsional rigidity (torsional resistance) of a pipe is explained below.

Fig. 8 shows a relationship between the deformation and the spacing between pipe cuts during bending with respect to torsional rigidity. In other words, the graphical representation of Fig. 8 shows the relationship between the deformation and the spacing between pipe cuts when the tube is twisted.

Fig. 8 shows the results of a twisting simulation of a pipe provided with cuts. The deformation of a tube provided with cuts during a twisting process is shown.

The dashed line indicates the spacing of a cut to its adjacent cut.

The solid line indicates the deformation of the pipe during twisting.

The ordinate and the abscissa each show length unit values (e.g. mm).

The following can be seen from Fig. 8: A pipe (or tube) has a certain torsional rigidity in its original form without cuts. As soon as this pipe is cut, the torsional rigidity decreases according to the shape and number of cuts. The greater the spacing between pipe cuts, the greater the torsional rigidity (and the smaller the deformation during rotation). If the spacing between pipe cuts becomes infinite, the pipe reaches its original highest torsional rigidity.

Since a high torsional rigidity is required for an insertion tube of an endoscope, the spacing between pipe cuts should consequently be as large as possible.

Fig. 9 shows, in a portion Y (region Y) in an enlarged section, the spacing (distance) M of each secondary cut 99 from its adjacent secondary cut 99 associated with the next main cut 98.

The structure in the portion Y shows that the spacing M between adjacent secondary cuts 99 should be as large as possible in order to provide a high degree of torsional rigidity. The exact spacing M between adjacent secondary cuts 99 can be determined according to individual needs.

The process of achieving dimensional stability (form/shape stability) of the pipe 2 is explained below.

A hard pipe is inherently dimensionally stable. The structure of the portion Y is configured such that the pipe 2 maintains dimensional stability after a plurality of cuts 98, 99 have been provided thereon.

In this case, the secondary cuts 99 are arranged spaced apart so far such that the portion Y is relatively long in the longitudinal direction of the pipe 2. In other words, this results in a wide annular portion in the portion Y which is free of cuts.

The portion Y can be considered as a short pipe (or tube) and therefore has a high degree of dimensional stability. If the entire pipe 2 is bent, sections F1, F2, F3 and F4 will yield because portion Y has inherent stability.

The pipe 2 is thus flexible in bending and at the same time dimensionally stable.

The interaction of the portions X and Y is explained below.

The overall structure of the proximal passive flexible section C is a combination between the portions X and Y.

Each of these portions X and Y provides a particular property to the pipe 2.

In the portion X, the main cuts 98 and secondary cuts 99 are arranged close to each other to achieve a high degree of flexibility.

In contrast, in the portion Y, the secondary cuts 99 are spaced further apart from each other to achieve a high degree of torsional rigidity.

This results in the following interactions between the portion X and the portion Y:
In the portion Y, the secondary cuts 99 are spaced far apart from each other. This portion Y is thus stable during both bending and twisting. During bending, the portion Y remains almost unchanged. The portion X, on the other hand, gives way and defines the flexibility of the entire pipe 2. The effect of the portion Y to the flexibility of the pipe 2 is insignificant.

In the portion X, the main cuts 98 and secondary cuts 99 are arranged very close to each other.

In the example, the main cuts 98 and the secondary cuts 99 have a different cut width with respect to each other. The cut width refers to the width of the respective cut in the longitudinal direction of the pipe. When the main cuts 98 and the secondary cuts 99 are formed by laser, the cut width is set by the choice of the diameter of the emitted laser beam bundle.

The cut width of the secondary cuts 99 should be kept as small as possible. By means of a laser, a cut width of, for example, far less than 20 µm can be achieved. For example, the secondary cuts 99 can be formed with a cut width of 20 µm. The main cuts 98 may be formed, for example, with a cut width of 0.2 mm. These values of the cut width constitute examples only. The appropriate cut widths in each case can be determined by tests.

Preferably, the cut width of the main cuts 98 is greater than the cut width of the secondary cuts 99. For example, the cut width of the main cuts 98 may be ten times the cut width of the secondary cuts 99. Again, this value is merely an example. The appropriate factor in each case may be set as needed. The disclosure is not limited to these values.

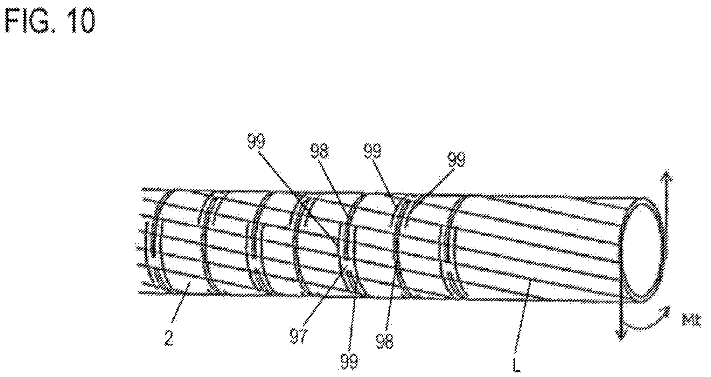

In the case of a torsional load, the pipe 2 is subject to a torsional moment (torsional torque) Mt acting about the longitudinal axis of the pipe 2. Due to the exertion of the torsional moment, imaginary (virtual) longitudinal lines L of the pipe 2 running parallel to the longitudinal axis deform helically (spirally), as shown in Fig. 10. Since the spacing N of the main cuts 98 and secondary cuts 99 in the portion X is very small, the deformation of the portion X will be only slightly different from that of the portion Y. The torsional rigidity of the portion Y defines the torsional rigidity of the entire pipe 2. The effect of the portion X to the torsional rigidity of pipe 2 is insignificant.

By forming cuts with different spacings from each other as explained above, both a high degree of flexibility and a high degree of torsional rigidity can be achieved in the proximal passive flexible section C of the pipe 2.

Thus, the endoscope tube 2 according to the disclosure in the proximal passive flexible section C of the flexible section 20 is bendable laterally to its longitudinal axis with a high degree of flexibility and also with a high degree of torsional rigidity.

The individual zones B, C and D in the flexible section 20 differ in that the spacing H of the cuts 98 in the longitudinal direction and thus the density of the cuts 98 are different.

In the zone B, the spacing H of the cuts 98 is the smallest. Thus, in the zone B, the density of the cuts 98 is the highest.

In the zone C, the spacing H of the cuts 98 is greater than in the zone B. In the zone D, the spacing H of the cuts 98 is greater than in the zone C.

Thus, the flexibility and bendability in the zone B is higher than in the zone C. Further, the flexibility and the bendability in the zone C is higher than in the zone D. In other words, the flexibility and the bendability of the respective zones of the flexible section 20 decrease in the proximal direction.

The zone D is provided with a portion on the proximal side that is not provided with cuts. This portion forms a transition to the control body J.

Transition from the bending section A to the flexible section 20

The transition portion from the bending section A to the flexible section 20 is indicated as portion/region K in Fig. 2. In this portion K, the bending section A ends. In other words, the first, i.e. most proximal, member of the bending section A is arranged distally of the portion K.

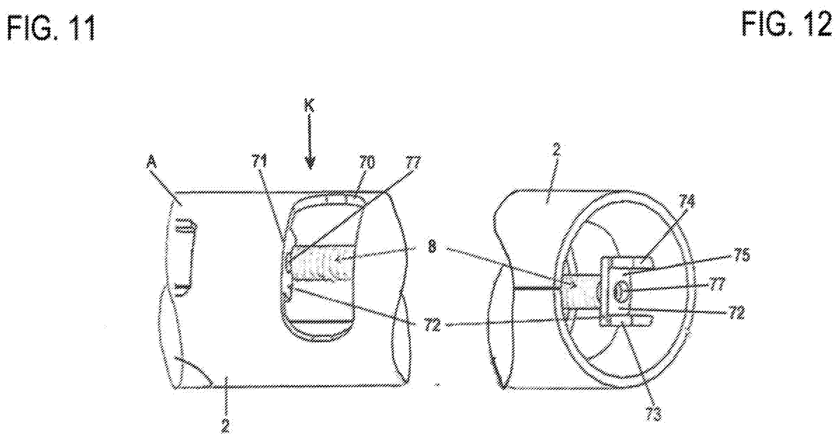

As shown in Figures 2, 11 and 12, in this portion K, the wall surface of the pipe element is cut by a cut 70 with the shape of an inverted letter C. In other words, the cut 70 in the pipe element is cut with the shape of an incomplete circle. The circle of the cut 70 is not cut through at the distal side, as can be seen from Fig. 11. The non-cut distal side of the cut 70 forms a hinge 71 for a flap (tab, clip) 72. The flap 72 has a lower ear 73, an upper ear 74 and a flap center piece 75. The lower ear 73 borders on an upper side of the flap center piece 75. The upper ear 74 borders on a lower side of the flap center piece 75.

The flap 72 is formed as follows. The location of the cut 70 is determined. A hole 77 is cut in the center of the cut 70. The cut 70 is formed by laser as shown in Fig. 2. The flap center piece 75 is supported from the rear side, i.e., from the inner side of the pipe element, by a post (or piston). The lower ear 73 is bent inwards relative to the flap center piece 75 by 90 degrees. The bending line of the ear 73 relative to the flap center piece 75 runs parallel to the axis of the pipe element (in Figures 2 and 4, in the direction pointing to the left and to the right). The upper ear 74 is also bent inwards relative to the flap centerpiece 75 by 90 degrees. The bending line of the ear 74 relative to the flap center piece 75 also runs parallel to the axis of the pipe element. Thereafter, the flap center piece 75 is bent inwards by 90 degrees. The bending line of the flap center piece 75 relative to the pipe element runs in the perpendicular sectional plane to the axis of the pipe element (in Figures 2 and 11, in the upward and downward direction). In other words, the flap center piece 75 is bent inwards by 90 degrees at the hinge 71. In particular, the flap center piece 75 is bent inwards so far until a distal side edge of the lower ear 73 and a distal side edge of the upper ear 74 abut the inner circumference of the pipe element, see Fig. 12.

The flap 72 serves as a support for a guide spring 8. In particular, the proximal surface of the flap center piece 75 forms a stop surface for the distal end of the guide spring 8. The two ears 73, 74 support the flap center piece 75 and absorb compressive forces acting from the guide spring 8 and transmit them to the inner circumferential surface of the pipe element.

The flap center piece 75 has the centric hole 77. The hole 77 has a larger diameter than a control wire and a smaller diameter than the guide spring 8. The control wire is guided in the flexible section 20 in the guide spring 8 and passes through the hole 70 and extends further into the bending section A.

In the portion K, flaps 72 are provided equal to the number of control wires used (four in the present example). The flaps 72 are evenly distributed in the circumferential direction of the pipe element.

### Bending section A

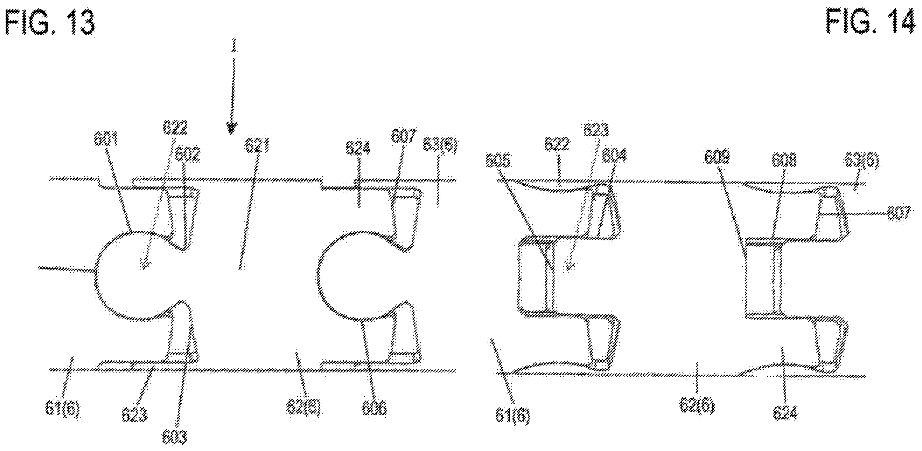

The detailed structure of the bending section A is shown in Figures 13 to 18.

The bending section A has individual joint members (articulating members, hinge members) 6 arranged in the longitudinal direction of the bending section A. The individual joint members 6 are pivotable relative to each other. In Figures 13 and 14, three consecutively arranged joint members 6 are shown: a joint 61, proximal to the joint 61 a joint 62, and proximal to the joint 62 a joint 63.

The joint members 6 are configured identically to each other with the exception of the most distal joint member 6 and the most proximal joint member 6.

The structure of the respective joint member 6 is discussed below with reference to joint member 62.

The joint member 62 is formed as a pipe segment (tube segment) of the pipe element by laser cutting. The joint member 62 has distal boundary lines 601, 602, 603, 604, and 605 and proximal boundary lines 606, 607, 608, and 609 at the circumference of the pipe element.

The individual distal boundary lines are made up of a circle-like shaped head line 601, two neck lines 602, two shoulder lines 603, two arm lines 604, and an arm end line 605. More specifically, the distal side of the joint member 62 is formed as follows. The circle-like shaped head line 601 forms an incomplete circle, which merges into a neck line 602 at the proximal side on each side. To each of the two neck lines 602, a shoulder line 603 connects, which extends approximately perpendicular to the axis of the pipe element. To each of the two shoulder lines 603, an arm line 604 connects, which extends approximately parallel to the axis of the pipe element in the distal direction. The two distal ends of the arm lines 604 are joined by an arm end line 605, which again extends perpendicular to the axis of the pipe element.

As a result, the joint member 62 has a main body 621 from which, toward the distal side, a first head 622, a first arm 623, a second head 622, and a second arm 623 protrude each by 90 degrees along an imaginary circumferential line that extends perpendicular to the axis of the joint member 62. Thus, the heads 622, 622 extend in a first imaginary plane. The arms 623, 623 extend in a second imaginary plane that is offset by 90 degrees from the first imaginary plane. The two heads 622, 622 of the joint member 62 form a pivoting axis for the joint member 61 arranged distally thereof.

Each head 622 is formed on the distal side by a head line 601. Between the head 622 and the main body 621, a constriction is formed by the neck lines 602. The respective head 622 projects further in the distal direction than the respective arm 623.

The individual proximal boundary lines are made up of a curved foot line 606, two bottom lines 607, two straight foot lines 608, and a waist line 609. More specifically, the proximal side of the joint member 62 is formed as follows. The curved foot line 606 forms an incomplete circle that is open at the proximal side. At each of the open ends of the incomplete circle, the curved foot line 606 merges with the bottom line 607, each of which extends approximately perpendicular to the axis of the pipe element.

Each of the two bottom lines 607 connects to a straight foot line 608, which extends approximately parallel to the axis of the pipe element in the distal direction. The two distal ends of the straight foot lines 608 are joined by a waist line 609, which again extends perpendicular to the axis of the pipe element.

As a result, the joint member 62 has two feet 624 at the proximal side of the main body 621, which extend in the proximal direction. Each foot 624 has, in the direction of extension, a straight side at the straight foot line 608 and a curved side at the curved foot line 606.

In the region between the two straight foot lines 608, an arm of the proximally located joint member 63 is arranged slidably in the longitudinal direction. In the region between the two curved foot lines 606, a head of the proximally located joint member 63 is held immovable in the longitudinal direction. At most, a slight movement due to a play between the inner circumference of the curved foot line and the outer circumference of the circle-like shaped head line is possible.

In the non-bent state of the bending section A, the waist line 609 is spaced apart from the arm end line 605 of the proximally located joint member 63, as shown in Fig. 14. The arm end line 605 and the waist line 609 of the proximally located joint member 63 are parallel to each other.

In the non-bent state of the bending section A, the bottom line 607 is spaced apart from the shoulder line 603 of the proximally located joint member 63, as shown in Fig. 14. The bottom line 607 and the shoulder line 603 of the proximally located joint member 63 may be parallel to each other or approximately parallel to each other, or may be slightly angled (tilted) with respect to each other, as shown in Fig. 14. Between the bottom line 607 and the shoulder line 603 of the proximally located joint member 63, not only a simple cut line was formed, but the material of the pipe element has been cut out as a four-sided piece.

A respective head 622 forms a coupling portion that is coupled to an adjacent joint member 6. The feet 624 form a guide portion that engages an adjacent joint member 6 so as to allow for an axial movement of the joint members 6 relative to each other.

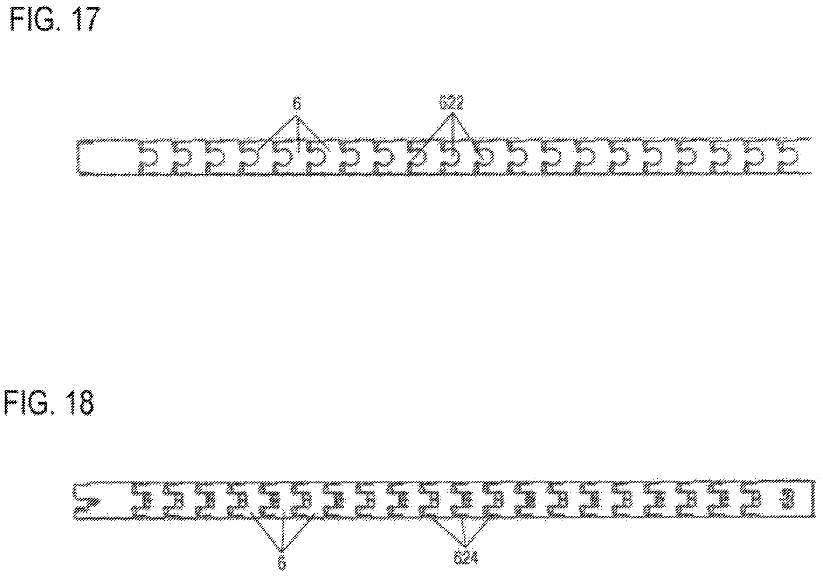

Fig. 17 shows a top view of the bending section A with the respective joint members 6. In the top view, the heads 622 of the joint members 6 can be seen.

Fig. 18 shows a side view of the bending section A with the respective joint members 6. In the side view, the feet 624 of the joint members 6 can be seen.

The most distal joint member 6 has no head and is shown in Figures 2 and 17 to 21.

The most proximal joint member 6 has no foot and is shown in Figures 2, 11 and 18.

In the example, the bending section A can be bent in two bending directions (angulation directions, deflection directions), namely upward and downward in Figs. 13 and 14 (and Fig. 17), wherein the respective heads 622 of the joint members 6 form bending axes of the joint members 6. In other words, the bending section A in Fig. 17 is pivotable upward and downward. In the illustration of Fig. 18, the bending section A is pivotable toward and away from the viewer.

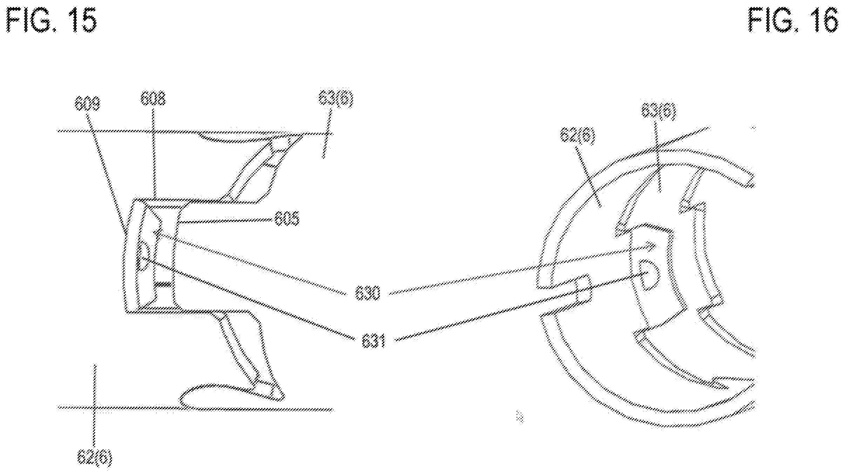

As shown in Figures 15 and 16, the waist line 609 forms a hinge portion for a cable guide flap (cable guide tab) 630. The cable guide flap 630 extends from the waist line 609. For the cable guide flap 630, a portion of material is taken that extends along the straight foot lines 608 to the arm end line 605 of the proximally located joint member 63. The cable guide flap 630 is hinged (articulated) at the waist line 609 and is bent inward by 90 degrees. The cable guide flap 630 has a centric hole 631. The hole 631 has a larger diameter than the control wire.

Each of the joint members 6 has the cable guide flaps 630 with the hole 631 such that the cable guide flaps 630 for a particular control wire are arranged consecutively in the longitudinal direction of the bending section A. The cable guide flaps 630 serve as guide protrusions on which a control wire is supported. Thus, the cable guide flaps 630 guide the control wire associated thereto through the bending section A.

The joint members 6 may be arranged at the bending section A such that their heads face in the proximal direction, as shown in Fig. 17. Alternatively, the joint members 6 may be arranged at the bending section A such that their heads face in the distal direction, as indicated in Fig. 13.

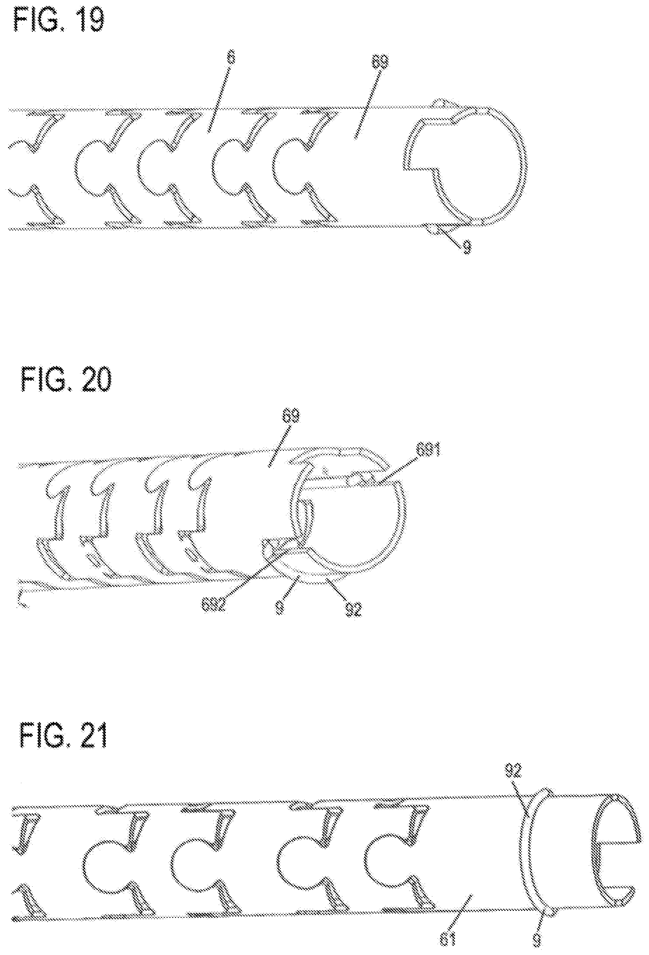

The distal end of the bending section A is shown in Figs. 19 to 21. In Figs. 19 to 21, the joint member 69 of the bending section A located furthest on the distal side can be seen. The distal side of the control wire 9 is anchored in this joint member 69 located furthest on the distal side. The control wire 9 extends from the control body 3 to the joint member 69 of the bending section A that is located furthest on the distal side.

### Fastening the control wire

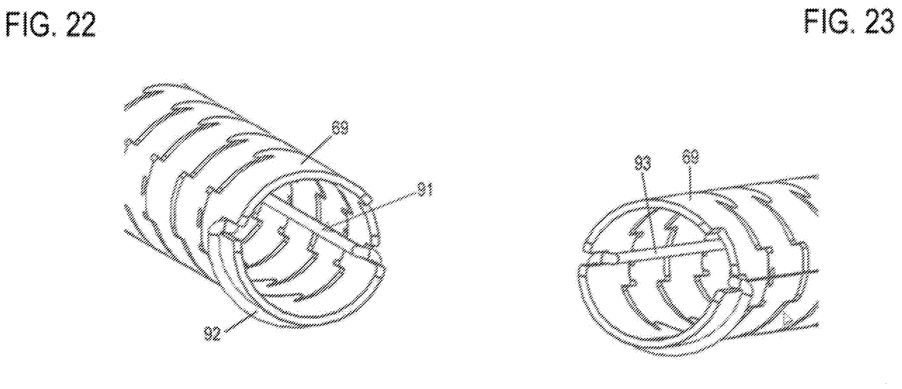

The attachment of the control wire 9 is shown in detail in Figures 22 and 23.

The control wire 9 is attached to the control wheel G in the control body 3. When the control wheel G is turned in a tensioning direction, the control wire 9 is tensioned. When the control wheel G is turned in the relieving direction opposite to the tensioning direction, the control wire 9 is relieved.

The control wire 9 extends from the control body 3 running in the insertion tube 2 to the joint member 69 and forms a first section 91. This first section 91 of the control wire 9 runs at the inner circumference of the insertion tube 2. This first section 91 of the control wire 9 is shown by reference sign 91 in Fig. 22. A slit 691 is formed at the distal side of the joint member 69 (see Fig. 20), the slit 691 penetrating the circumferential wall of the joint member 69 and extending in the longitudinal direction of the joint member 69. Another similar slit 692 is provided at the distal side of the joint member 69 diametrically opposite the slit 691.

The control wire 9 extends in the distal direction at the inner circumference of the joint member 69 and extends through the slit 691 to the outside, is wound in the circumferential direction of the joint member 69 up to the slit 692 at the outer circumference of the joint member 69, extends through the slit 692 to the inside, and extends in the proximal direction at the inner circumference of the joint member 69 up to the control wheel G in the control body 3.

The control wire 9 is thus divided into a first section 91 extending from the control wheel G in the control body 3 to the slit 691, a second section 92 extending from the slit 691 at the outer circumference of the joint member 69 in the circumferential direction of the joint member 69 to the slit 692, and a third section 93 extending from the slit 692 to the control wheel G in the control body 3.

By turning the control wheel G in the tensioning direction, the control wire 9 is tensioned and thereby the bending section A is bent since the third portion 93 anchored at the joint member 69 is urged in the proximal direction. The third portion 93 of the control wire 9 thus forms a distal anchoring portion of the control wire 9.

### Manufacturing method

The insertion tube 2 according to the disclosure can be manufactured from a single pipe element, which is cut by laser. The pipe element is made of a relatively hard material, such as stainless steel or even suitable hard plastic. As a result of the cuts, the initially hard pipe element becomes flexible but retains its stiffness.

The cuts form the respective lateral incisions (cuts running perpendicular to the axis) 98, 99 in the proximal passive flexible section 20, the hole 77, the cut 70 in the transition portion K, the hole 631, the respective joint members 6 in the distal bending section A, and the slits 691, 692. This order is not to be construed as a limitation. For example, the slits 691, 692 may be cut before the joint members 6. Furthermore, the order of the cuts may also be reversed.

The flexibility and also the stiffness of the pipe element can be controlled by the shape, the arrangement and the size of the cuts.

The location of the respective cuts can be calculated beforehand and be predetermined. In a programmable laser cutting machine, the specified data for the respective cuts can be entered to automatically form the insertion tube 2.

The individual joint members 6 are completely cut out and form physically separate bodies from each other, which are only form-fittingly connected (interlockingly connected, positively connected).

After laser cutting the pipe element, the flaps 72 and the cable guide flaps 630 are bent inwards. The raw body for the insertion tube 2 is thus completed.

The control wire 9 can now be inserted and attached in this raw body for the insertion tube 2. The raw body for the insertion tube 2 can be attached to the control body 3. Furthermore, a coating, preferably of metal for shielding the electrical control, surrounding the raw body for the insertion tube 2 can be fitted onto the raw body for the insertion tube 2 and an elastic cover (sheath) of plastic or rubber can be fitted onto the coating. The elastic cover of plastic or rubber can be subjected to thermal shrinkage.

### Second example

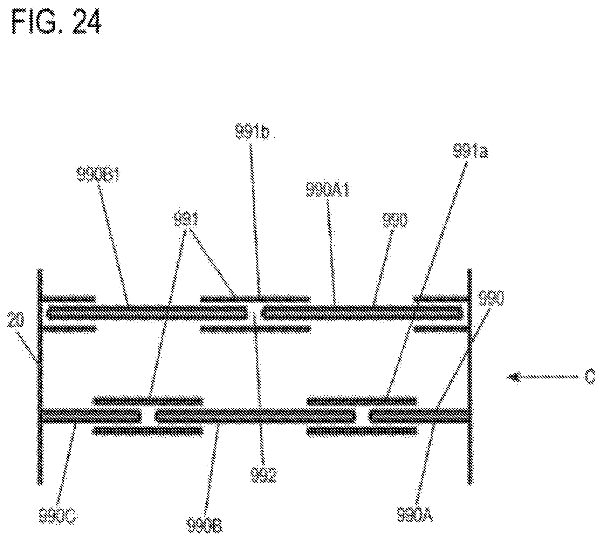

Referring now to Fig. 24, a second example of the present disclosure is described below.

Fig. 24 shows a partial schematic view of the proximal passive flexible section employed in the second example.

The proximal passive flexible section 20 constructed according to the principle illustrated in Fig. 24 may replace the proximal passive flexible section 20 of the first example. In other words, the control body 3 and the bending section A can be combined with the proximal passive flexible section 20 of the present second example.

As described above, the distal bending section A and the proximal passive flexible section 20 with the three zones B, C and D are formed from a single pipe or tube, see also Fig. 1.

The zone B forms a transition portion B between the middle portion C and the bending section A. The zone C forms the middle portion C. The zone D forms a connecting portion D of the proximal passive flexible section 20 at the control body 3. In other words, the entire insertion tube including the connecting portion D at the control body 3, the middle portion C, the transition portion B between the middle portion C and the bending section A, and the bending section A is made of a single pipe element.

Fig. 1 shows the proximal passive flexible section 20 for better clarity as if the three zones B, C and D were of equal length to each other along the longitudinal direction of the insertion tube 2. This is, of course, not the case. The middle portion C is longer than the transition portion B and the connecting portion D. The middle portion C is the longest in the proximal passive flexible section 20. In other words, the actual proximal passive flexible section 20 is formed by the structure of the middle portion C. The bending properties, elasticity and torsional rigidity of the proximal passive flexible section 20 are implemented by the structure of the middle portion C.

The structure of the middle portion C of the proximal passive flexible section 20 is described in more detail below with reference to Fig. 24.

The proximal passive flexible section 20 is made of the pipe element already described above. In the central portion C, a plurality of main cuts 990 are cut by laser cutting along the longitudinal direction of the pipe element. These main cuts 990 extend parallel to each other. The main cuts 990 extend perpendicular to the axis of the pipe element.

More specifically, the main cuts 990 extend along the circumference of the central portion C in an interrupted manner such that uncut bridges (stays) 992 remain between main cut portions lying on a circumferential line. In the present example, four main cut portions are formed as viewed in the circumferential direction.

Fig. 24 shows these main cut portions in more detail. Fig. 24 shows a first sequence of main cut portions formed in the circumferential direction with reference numerals 990A, 990B and 990C. Fig. 24 further shows a second sequence of main cut portions formed in the circumferential direction with reference numerals 990A1 and 990B1. The first sequence of main cut portions with the reference signs 990A, 990B and 990C is adjacent in the longitudinal direction to the second sequence of main cut portions formed in the circumferential direction with the reference signs 990A1 and 990B1. The length of the main cut portions in the circumferential direction is always the same. That is, not only the length of the main cut portions in the circumferential direction of a particular sequence of main cut portions is equal to each other, but the length of the main cut portions in the circumferential direction of all sequences of main cut portions in the entire middle portion C is equal to each other.

In the first sequence of main cut portions shown in Fig. 24, a first main cut portion 990A, a second main cut portion 990B, and a third main cut portion 990C are shown. A fourth main cut portion, which is not visible, is arranged on the side of the pipe element facing away from the viewer, behind the drawing plane. The first main cut portion 990A, the second main cut portion 990B, the third main cut portion 990C, and the fourth main cut portion not shown are formed consecutively in the circumferential direction of the pipe element. Thus, at this circumferential line, the pipe element is cut sectionally four times with the same length. A respective bridge 992 is left between an end of the first main cut portion 990A and a beginning of the second main cut portion 990B, an end of the second main cut portion 990B and a beginning of the third main cut portion 990C, an end of the third main cut portion 990C and a beginning of the fourth main cut portion not shown, and an end of the fourth main cut portion not shown and a beginning of the first main cut portion 990A. The pipe element is not cut in the region of the bridge 992.

In the second sequence of main cut portions shown in Fig. 24, a first main cut portion 990A1 and a second main cut portion 990B1 are shown. A third main cut portion and a fourth main cut portion that are not visible are arranged on the side of the pipe element facing away from the viewer, behind the drawing plane.

The main cut portions of the second sequence are offset relative to the main cut portions of the first sequence. In the adjacent second sequence, the region of the first sequence where the main cut portions 990A, 990B and 990C leave the respective bridge 992 corresponds to a region that forms the center of the main cut portions 990A1 and 990B1 as viewed in the circumferential direction of the pipe element. Thus, the bridges are positioned offset by 45 degrees from sequence to sequence of main cuts 990 in the longitudinal direction of the pipe element.

The cut width of all main cuts 990 in the pipe element is the same. The spacing of all sequences of main cuts 990 in the pipe element is the same.

In the longitudinal direction of the pipe element, a secondary cut 991 is provided adjacent to each bridge 992, as shown in Fig. 24.

A secondary cut 991 is formed adjacent to the bridge 992 on both sides in the longitudinal direction of the pipe element. The secondary cut 991 is shorter than the main cut 990. The secondary cut 991 overlaps with the ends of the adjacent main cuts 990.

All of the secondary cuts 991 have the same length relative to each other in the circumferential direction of the pipe element. All secondary cuts 991 are parallel to each other and also parallel to the main cuts 990.

Adjacent to both sides in the longitudinal direction of the pipe element, a respective sequence of secondary cuts 991 each is associated with a sequence of main cuts 990. In other words, each sequence of main cuts 990 has a proximal sequence of secondary cuts 991 and a distal sequence of secondary cuts 991.

Thus, seen along the longitudinal direction of the pipe element, a sequence of main cuts 990 is followed by a distal sequence of secondary cuts 991, which is again followed by a proximal sequence of secondary cuts 991 of the next sequence of main cuts 990. Viewed along the longitudinal direction of the pipe element, a sequence of secondary cuts 991 has a further sequence of secondary cuts 991 as a neighbor on one side and a sequence of main cuts 990 as a neighbor on the other side.

The secondary cuts 991 are formed closer in the longitudinal direction of the pipe element to the nearest main cuts 990 than to the nearest secondary cuts 991.

In other words, adjacent to the main cuts 990, secondary cuts 991 are provided such that they are arranged closer to the adjacent main cuts 990 than to the adjacent secondary cuts 991.

To illustrate this, Fig. 24 shows the secondary cuts 991 for the first sequence of main cut portions as secondary cuts 991a and the secondary cuts 991 for the second sequence of main cut portions as secondary cuts 991b. The secondary cuts 991a for the first sequence of main cut portions are arranged closer to the adjacent main cut portions 990A, 990B and 990C than to the adjacent secondary cuts 991b. Thus, adjacent cuts in the pipe element are unequally spaced.

The cut width of all secondary cuts 991 in the pipe element is the same. The cut width of the secondary cuts 991 is narrower than the cut width of the main cuts 990.

### Effect of the second example

As in the first example, the structure of the second example provides an insertion tube 2 with a very high degree of flexibility and at the same time a high degree of torsional rigidity.

### Third example

In the first and second examples, in the flexible section C, the main cuts are equally spaced from each other.

In contrast, in the present third example, in the flexible section C, the main cuts are unequally spaced from each other. The other aspects are the same as in the previous examples.

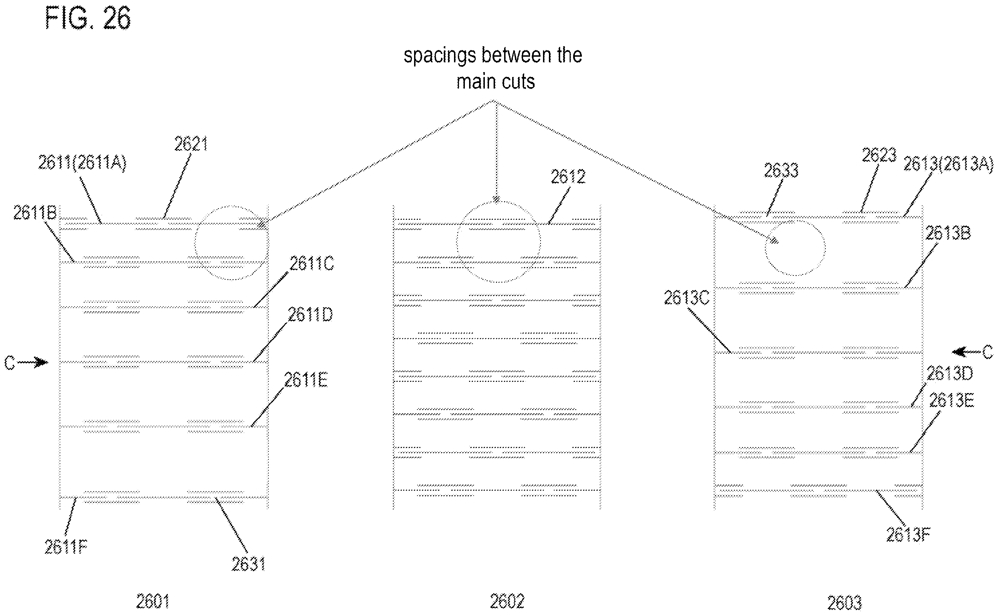

Fig. 26 shows a partial schematic view of the proximal passive flexible section in a third example in two variants in comparison with the previous examples.

In particular, Fig. 26 shows a first variant 2601 of the cut design in the flexible section C; a second variant 2602 of the cut design in the flexible section C; and a third variant 2603 of the cut design in the flexible section C.

In the second variant 2602, as in the first and second examples, the main cuts 2612 are equally spaced from each other in the flexible section C for comparison purposes.

In the first variant 2601 and in the third variant 2603, however, adjacent main cuts 2611, 2613 in the flexible section C are not equally spaced from each other. Fig. 26 shows a subzone in the proximal passive flexible section C in each of the first variant 2601 and the third variant 2603. In this subzone, the spacing between adjacent main cuts is unequal.

In the respective variants shown, the spacings (distances) between the adjacent main cuts are thus designed differently. In the respective upper section of figure 26, a spacing between adjacent main cuts is marked by means of a circle. In the distal direction of the flexible section C, this spacing between adjacent main cuts increases in the first variant 2601; remains the same in the second variant 2602; and decreases in the third variant 2603.

The first variant 2601 shows the case in which the spacings - measured in the direction of extension of the endoscope - between the adjacent main cuts 2611 increase towards the distal side. The main cuts shown in the first variant 2601 are grouped together using the reference sign 2611. Non-cut bridges (stays) 2631 are present between main cut portions 2611 lying on a circumferential line. Secondary cuts are shown with reference sign 2621. The secondary cuts are described in the previous examples. Express reference is made to the details explained therein.

A first main cut 2611A shown is spaced from the second main cut 2611B shown by a spacing that is smaller than a spacing between the second main cut 2611B shown and a third main cut 2611C shown. The spacing between the second main cut 2611B shown and the third main cut 2611C shown is in turn smaller than a spacing between the third main cut 2611C shown and a fourth main cut 2611D shown, and so on. In the distal direction, the spacing between the main cuts 2611A, 2611B, 2611C, 2611D, 2611E, and 2611F shown becomes larger and larger.

In the first variant 2601, the spacing between the main cuts may increase uniformly (continuously) towards the distal side.

For example, the increase in spacing may be such that the second main cut 2611B is spaced from the third main cut 2611C by a spacing H2 that is greater than a spacing H1 between the first main cut 2611A and the second main cut 2611B by a value y (difference); and the fourth main cut 2611D is spaced from the third main cut 2611C by a spacing H3 that is greater than the spacing H2 by the same value y.

In another example, the increase in spacing may be such that the second main cut 2611B is spaced from the third main cut 2611C by a spacing H2 that is greater than a spacing H1 between the first main cut 2611A and the second main cut 2611B by a value y; and the fourth main cut 2611D is spaced from the third main cut 2611C by a spacing H3 that is greater than the spacing H2 by y multiplied by a factor z (which is greater than 1).

The increase in spacing can be embodied arbitrarily.

In the first variant 2601, the spacing between the main cuts may also increase unevenly (discontinuously) towards the distal side.

The third variant 2603 shows the case in which the spacings - measured in the direction of extension of the endoscope - between the adjacent main cuts 2613 decrease towards the distal side. The main cuts shown in the third variant 2603 are grouped together using the reference sign 2613. Non-cut bridges 2633 are present between main cut portions 2613 lying on a circumferential line. Secondary cuts are shown with reference sign 2623. The secondary cuts are described in the previous examples. Express reference is made to the details explained therein.

A first main cut 2613A shown is spaced from the second main cut 2613B shown by a spacing that is greater than a spacing between the second main cut 2613B shown and a third main cut 2613C shown. The spacing between the second main cut 2613B shown and the third main cut 2613C shown is in turn greater than a spacing between the third main cut 2613C shown and a fourth main cut 2613D shown, and so on. In the distal direction, the spacing between the main cuts 2613A, 2613B, 2613C, 2613D, 2613E, and 2613F shown becomes smaller and smaller.

In the third variant 2603, the spacing between the main cuts may decrease uniformly (continuously) towards the distal side. However, in the third variant 2603, the spacing between the main cuts may also decrease towards the distal side unevenly (discontinuously). As in the first variant, the increase in spacing can be embodied arbitrarily.

Thus, in the first variant 2601, the main cuts 2611 are spaced from each other in the longitudinal direction of the proximal passive flexible section C with continuously increasing spacing; and in the third variant 2603, the main cuts 2613 are spaced from each other in the longitudinal direction of the proximal passive flexible section C with continuously decreasing spacing.

The first variant 2601 and the third variant 2603 may be combined as subzones in a proximal passive flexible section C such that, at least within a first subzone 2601 in the proximal passive flexible section C, the main cuts 2611 are spaced from each other in the longitudinal direction of the proximal passive flexible section C with continuously increasing spacing, and at least within a second subzone 2603 in the proximal passive flexible section C, the main cuts 2613 are spaced from each other in the longitudinal direction of the proximal passive flexible section C with continuously decreasing spacing.

The first subzone 2601 and the second subzone 2603 may border (abut) on each other.

In another example, a third subzone 2602 may be present between the first subzone 2601 and the second subzone 2603, the third subzone 2602 with an equal spacing between the main cuts.

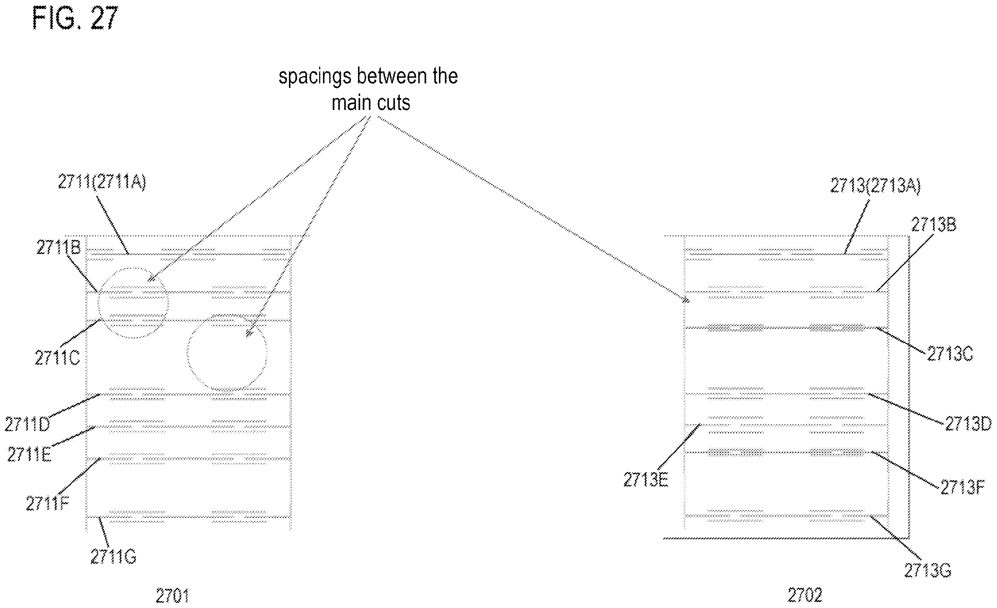

Fig. 27 shows a partial schematic view of the proximal passive flexible section in the third example in further variants.

In the examples of Figure 26, a decrease or increase of the spacings between the adjacent main cuts is shown. The disclosure is not limited thereto.

Adjacent main cuts may also have unequal spacing with respect to each other without the spacing between the adjacent main cuts increasing or decreasing in the direction of extension of the endoscope.

Fig. 27 shows an example of this.

Fig. 27 shows on the left an example of a fourth variant as subzone 2701.

Fig. 27 shows on the right side an example of a fifth variant as subzone 2702.

In the two variants shown, the spacings between the adjacent main cuts are also designed differently. In Figure 27, a spacing between adjacent main cuts is marked by means of a circle.

The main cuts shown in the fourth variant 2701 are grouped together using reference sign 2711. The main cuts in the fifth variant 2702 are grouped together using the reference sign 2713. Non-cut bridges (without reference signs) are present between main cut portions lying on a circumferential line. Secondary cuts are shown without reference signs. The secondary cuts are described in the previous examples. Express reference is made to the details explained therein.

In the fourth variant 2701, a first main cut 2711A shown is spaced from the second main cut 2711B shown by a spacing that is greater than a spacing between the second main cut 2711B shown and a third main cut 2711C shown. The spacing between the second main cut 2711B shown and the third main cut 2711C shown is smaller than a spacing between the third main cut 2711C shown and a fourth main cut 2711D shown. The spacing between the third main cut 2711C shown and the fourth main cut 2711D shown is greater than a spacing between the fourth main cut 2711D shown and a fifth main cut 2711E shown. The spacing between the fourth main cut 2711D shown and a fifth main cut 2711E shown is approximately equal to a spacing between the fifth main cut 2711E shown and a sixth main cut 2711F shown. The spacing between the fifth main cut 2711E shown and the sixth main cut 2711F shown is smaller than a spacing between the sixth main cut 2711F shown and a seventh main cut 2711G shown.

Thus, in the distal direction, there is a rather particular respective spacing between the main cuts 2711A, 2711B, 2711C, 2711D, 2711E, 2711F and 2711G shown.

In the fifth variant 2702, also a particular respective spacing between the main cuts 2713A, 2713B, 2713C, 2713D, 2713E, 2713F and 2713G shown is illustrated.

In another variant not shown, adjacent main cuts may each have completely arbitrary spacings from each other. A relatively short spacing can follow a relatively large spacing.

Figures 26 and 27 each show a subzone in the proximal passive flexible section C. In this subzone, the spacing between adjacent main cuts is unequal. Bordering on this subzone, in the remaining part of the proximal passive flexible section C, the spacing between adjacent main cuts may be equal. Alternatively, in the entire proximal passive flexible section C, the spacing between adjacent main cuts may be unequal. Different ones of the possibilities shown may be suitably combined for designing unequal adjacent spacings between adjacent main cuts.

The third example thus shows an endoscope with an insertion tube 2, the insertion tube 2 having a proximal passive flexible section C and a distal bending section A, cuts 2611, 2621; 2613, 2623 being provided in the proximal passive flexible section C to allow for bending the proximal passive flexible section C, adjacent cuts 2611, 2621; 2613, 2623 in the proximal passive flexible section C being unequally spaced, the proximal passive flexible section C having secondary cuts 2621; 2623 adjacent to main cuts 2611; 2613, the secondary cuts 2621; 2623 being arranged closer in the longitudinal direction of the proximal passive flexible section C to the adjacent main cuts 2611; 2613 on one side of the secondary cuts 2621; 2623 than to the adjacent main cuts 2611; 2613 on the other side of the secondary cuts 2621; 2623, the main cuts 2611; 2613 extending along the circumference of the proximal passive flexible section C in an interrupted manner such that uncut bridges (stays) 2631; 2633 remain between main cut portions 2611; 2613 lying on a circumferential line, and the proximal passive flexible section C including the main cuts 2611; 2613, wherein at least within a subzone in the proximal passive flexible section C, the main cuts 2611; 2613 are unequally spaced from each other in the longitudinal direction of the proximal passive flexible section C.

### Effect of the third example

Due to the varying spacing between adjacent main cuts, a proximal passive flexible section C can be designed in which a highly flexible bending (angulation, deflection) can be achieved.

A larger spacing between adjacent main cuts results in a smaller bending angle and a smaller angulation range (bending range, deflection range) in the proximal passive flexible section C. A smaller spacing between adjacent main cuts results in a larger bending angle and a larger angulation range in the proximal passive flexible section C.

A structure with an ever increasing spacing between adjacent main cuts results in an ever decreasing bending angle and an ever decreasing angulation range in the proximal passive flexible section C.

A structure with an ever decreasing spacing between adjacent main cuts results in an ever increasing bending angle and an ever increasing angulation range in the proximal passive flexible section C.

By combining small and large spacings between adjacent main cuts, a highly individual design of the bending angle and the articulation range in the proximal passive flexible section C in the direction of extension becomes possible.

Bending shapes (angulation shapes, deflection shapes) not previously used in practice can be achieved, which are adapted to even complex anatomical conditions.

Certain selected and precisely defined sections and subsections in the bending section of the endoscope can be assigned certain bending properties (bending curve, stiffness, etc.) in a highly targeted manner.

### Other alternatives

In the first and second examples, the flexible section 20 has a first zone B, a second zone C, and a third zone D with different flexibility when viewed in the proximal direction. The number of zones or portions with different flexibility is not limited. The flexible section 20 may also have more or less zones with different flexibility. The disclosure is also applicable to an insertion tube in which the flexible section 20 has a constant flexibility throughout.

In the first and second examples, the pipe element of the insertion tube 2 is formed of stainless steel. The disclosure is not limited thereto. The material of the insertion tube 2 may be any sufficiently stiff material, such as a stiff plastic. In another alternative, nitinol (a nickel-titanium alloy) may be employed as the pipe material. This material has, among other things, the property of so-called superelasticity, i.e. it can be elastically deformed over wide ranges without being bending permanently.

In the first and second examples, cuts are provided in the pipe element by a laser cutting machine. These cuts can be provided very precisely. Therefore, manufacturing by laser is preferred. However, in principle it is conceivable that these cuts can also be fabricated by other manufacturing methods such as sawing, wire sawing, etc.

In the first and second examples, the bending section A can be bent (angled, deflected) in two bending directions (angulation directions), namely upward and downward in Figures 6 and 7. In an alternative, the individual joint members 6 may be formed such that their heads 622 from joint member 6 to joint member 6 are offset rotated by 90 degrees about the axis of the bending section A (axis of the joint members 6). In this alternative, the bending section A can be bent in four bending directions, namely up and down and towards and away from the viewer in Figures 6 and 7.

In the alternative, in which the bending section A can be bent in four bending directions, two control wires 9 can be used which extend in the insertion tube 2 offset by 90 degrees from each other. The joint member 92 is then provided with four distal slits which are also offset by 90 degrees from each other.

In the example, a respective joint member 6 is formed in the shape described. The disclosure is not limited to the shape of the joint member 6. It is sufficient if joint members are cut in the bending section A that are coupled to each other and allow for a deflecting movement of the bending section A.

The proximal passive flexible section C constructed according to the principle illustrated in Fig. 24 may be applied in the first or second example. This means that the proximal passive flexible section C shown in Fig. 24 forms part of the single-piece pipe element for the entire insertion tube 20. Thus, the pipe element for the entire insertion tube 20 including the proximal passive flexible section C is made of/from a pipe element by laser cutting.

Alternatively, the proximal passive flexible section C may be manufactured separately from the rest of the insertion tube 20 in the first or second example.

In the example of Fig. 24, in the longitudinal direction of the pipe element, two secondary cuts are arranged adjacent to each bridge on both sides of the bridge. In an alternative, in the longitudinal direction of the pipe element, one secondary cut may be arranged adjacent to each bridge on one side of the bridge.

In the first example, the main cuts are provided such that two bridges remain between the main cut portions along the circumference of the pipe element.

In the second example, the main cuts are provided such that four bridges remain between the main cut portions along the circumference of the pipe element.

The disclosure is not limited thereto. Preferably, the number of bridges along the circumference of the pipe element between the main cut portions is at least two or more and may be any number.

In the first example, the cut width of the main cuts 98 is greater than the cut width of the secondary cuts 99. Also in the second example, the cut width of the main cuts may be greater than the cut width of the secondary cuts. However, the principle of the disclosure is also applicable in the case where the cut width of the main cuts is equal to the cut width of the secondary cuts.

The disclosure can advantageously be employed in a duodenoscope, a gastroscope, a colonoscope or a similar endoscope. The principle of the disclosure can also be applied to any other type of endoscope.

The principle of the disclosure can also be employed in other medical devices that use an insertion tube.

## Claims

1. An endoscope with an insertion tube (2),
wherein the insertion tube (2) comprises a proximal passive flexible section (C) and a distal bending section (A),
cuts (2611, 2621; 2613, 2623) are provided in the proximal passive flexible section (C) to allow for bending the proximal passive flexible section (C),
adjacent cuts (2611, 2621; 2613, 2623) in the proximal passive flexible section (C) are unequally spaced,
the proximal passive flexible section (C) comprises secondary cuts (2621; 2623) adjacent to main cuts (2611; 2613), wherein the secondary cuts (2621; 2623) are arranged closer (N) in a longitudinal direction of the proximal passive flexible section (C) to the adjacent main cuts (2611; 2613) on one side of the secondary cuts (2621; 2623) than to the adjacent main cuts (2611; 2613) on the other side of the secondary cuts (2621; 2623), and
the main cuts (2611; 2613) extend along the circumference of the proximal passive flexible section (C) in an interrupted manner such that uncut bridges (2631; 2633) remain between main cut portions (2611; 2613) lying on a circumferential line,
wherein the proximal passive flexible section (C) comprises the main cuts (2611; 2613), wherein at least within a subzone in the proximal passive flexible section (C) the main cuts (2611; 2613) are unequally spaced from each other in the longitudinal direction of the proximal passive flexible section (C),
**characterized in that**
at least within a first subzone (2601) in the proximal passive flexible section (C), the main cuts (2611; 2613) are spaced from each other in the longitudinal direction of the proximal passive flexible section (C) with continuously increasing spacing, and
at least within a second subzone (2603) in the proximal passive flexible section (C), the main cuts (2611; 2613) are spaced from each other in the longitudinal direction of the proximal passive flexible section (C) with continuously decreasing spacing,
wherein
the first subzone (2601) and the second subzone (2603) border on each other, or
a third subzone (2602) is arranged between the first subzone (2601) and the second subzone (2603), wherein the main cuts (2612) are equally spaced from each other in the longitudinal direction of the proximal passive flexible section (C) in the third subzone (2602).

2. The endoscope according to claim 1, wherein
the main cuts (2611; 2613) are parallel to each other.

3. The endoscope according to claim 1 or 2, wherein
the secondary cuts (2621; 2623) are each arranged adjacent to a bridge (2631; 2633) between main cut portions lying on a circumferential line,
in particular wherein
one secondary cut (2621; 2623) is arranged adjacent to each bridge (2631; 2633) in the longitudinal direction of the proximal passive flexible section (C) on one side of the bridge (2631; 2633), or
two secondary cuts (2621; 2623) are arranged adjacent to each bridge (2631; 2633) in the longitudinal direction of the proximal passive flexible section (C) on both sides of the bridge (2631; 2633).

4. The endoscope according to any one of claims 1 to 3, wherein
the main cuts (2611; 2613) are wider than the secondary cuts (2621; 2623).

5. The endoscope according to any one of claims 1 to 4, wherein
the entire insertion tube (2) including a connecting portion (D) of the proximal passive flexible section (C) to a control body (3), the proximal passive flexible section (C), a transition portion (B) between the proximal passive flexible section (C) and the bending section (A), and the bending section (A) is made of a single pipe element.

6. The endoscope according to any one of claims 1 to 5, wherein
the entire insertion tube (2) is cut by laser.

7. A method of manufacturing an insertion tube (2) of an endoscope from a pipe element,
wherein the insertion tube (2) comprises a proximal passive flexible section (C) and a distal bending section (A),
wherein cuts (2611, 2621; 2613, 2623) are provided in the proximal passive flexible section (C) to allow for bending the proximal passive flexible section (C),
wherein:
the cuts (2611, 2621; 2613, 2623) in the proximal passive flexible section (C) are formed such that adjacent cuts (2611, 2621; 2613, 2623) are unequally spaced,
secondary cuts (2621; 2623) are provided in the proximal passive flexible section (C) adjacent to main cuts (2611; 2613), wherein the secondary cuts (2621; 2623) are arranged closer in a longitudinal direction of the proximal passive flexible section (C) to the adjacent main cuts (2611; 2613) on one side of the secondary cuts (2621; 2623) than to the adjacent main cuts (2611; 2613) on the other side of the secondary cuts (2621; 2623), and
the main cuts (2611; 2613) are cut along the circumference of the proximal passive flexible section (C) in an interrupted manner such that uncut bridges (2631; 2633) remain between main cut portions lying on a circumferential line,
wherein the main cuts (2611; 2613) are provided in the proximal passive flexible section (C), wherein at least within a subzone the main cuts (2611; 2613) are unequally spaced from each other in the longitudinal direction of the proximal passive flexible section (C),
**characterized in that**
at least within a first subzone (2601) in the proximal passive flexible section (C), the main cuts (2611; 2613) are spaced from each other in the longitudinal direction of the proximal passive flexible section (C) with continuously increasing spacing, and
at least within a second subzone (2603) in the proximal passive flexible section (C), the main cuts (2611; 2613) are spaced from each other in the longitudinal direction of the proximal passive flexible section (C) with continuously decreasing spacing (H),
wherein
the first subzone (2601) and the second subzone (2603) border on each other, or
a third subzone (2602) is arranged between the first subzone (2601) and the second subzone (2603), wherein the main cuts (2611; 2613) are equally spaced from each other in the longitudinal direction of the proximal passive flexible section (C) in the third subzone (2602).

8. The method according to claim 7, wherein
the main cuts (2611; 2613) are cut parallel to each other.

9. The method according to claim 7 or 8, wherein
the secondary cuts (2621; 2623) are each cut adjacent to a bridge (2631; 2633) between main cut portions lying on a circumferential line,
in particular wherein
one secondary cut (2621; 2623) is cut adjacent to each bridge (2631; 2633) in the longitudinal direction of the proximal passive flexible section (C) on one side of the bridge (2631; 2633), or
two secondary cuts (2621; 2623) are cut adjacent to each bridge (2631; 2633) in the longitudinal direction of the proximal passive flexible section (C) on both sides of the bridge (2631; 2633).

10. The method according to any one of claims 7 to 9, wherein
the main cuts (2611; 2613) are cut wider than the secondary cuts (2621; 2623).

11. The method according to any one of claims 7 to 10, wherein
the entire insertion tube (2) including a connecting portion (D) of the proximal passive flexible section (C) to a control body (3), the proximal passive flexible section (C), a transition portion (B) between the proximal passive flexible section (C) and the bending section (A), and the bending section (A) is made of a single pipe element, and/or
the entire insertion tube (2) is cut by laser.

## Patentansprüche

1. Endoskop mit einem Einführschlauch (2),
wobei der Einführschlauch (2) einen proximalen passiven flexiblen Abschnitt (C) und einen distalen Biegeabschnitt (A) aufweist,
Einschnitte (2611, 2621; 2613, 2623) in dem proximalen passiven flexiblen Abschnitt (C) vorgesehen sind, um ein Biegen des proximalen passiven flexiblen Abschnitts (C) zu ermöglichen,
benachbarte Einschnitte (2611, 2621; 2613, 2623) in dem proximalen passiven flexiblen Abschnitt (C) ungleichmäßig beabstandet sind,
der proximale passive flexible Abschnitt (C) sekundäre Einschnitte (2621; 2623) benachbart zu Haupteinschnitten (2611; 2613) aufweist, wobei die sekundären Einschnitte (2621; 2623) in einer Längsrichtung des proximalen passiven flexiblen Abschnitts (C) näher (N) zu den benachbarten Haupteinschnitten (2611; 2613) auf einer Seite der sekundären Einschnitte (2621; 2623) als zu den benachbarten Haupteinschnitten (2611; 2613) auf der anderen Seite der sekundären Einschnitte (2621; 2623) angeordnet sind, und
die Haupteinschnitte (2611; 2613) sich entlang des Umfangs des proximalen passiven flexiblen Abschnitts (C) in einer unterbrochenen Weise erstrecken, so dass nicht eingeschnittene Brücken (2631; 2633) zwischen Haupteinschnittteilen (2611; 2613) verbleiben, die auf einer Umfangslinie liegen,
wobei der proximale passive flexible Abschnitt (C) die Haupteinschnitte (2611; 2613) aufweist, wobei zumindest innerhalb einer Teilzone in dem proximalen passiven flexiblen Abschnitt (C) die Haupteinschnitte (2611; 2613) in der Längsrichtung des proximalen passiven flexiblen Abschnitts (C) ungleichmäßig voneinander beabstandet sind,
**dadurch gekennzeichnet, dass**
zumindest innerhalb einer ersten Teilzone (2601) in dem proximalen passiven flexiblen Abschnitt (C) die Haupteinschnitte (2611; 2613) in der Längsrichtung des proximalen passiven flexiblen Abschnitts (C) mit kontinuierlich zunehmendem Abstand voneinander beabstandet sind, und
zumindest innerhalb einer zweiten Teilzone (2603) in dem proximalen passiven flexiblen Abschnitt (C) die Haupteinschnitte (2611; 2613) in der Längsrichtung des proximalen passiven flexiblen Abschnitts (C) mit kontinuierlich abnehmendem Abstand voneinander beabstandet sind,
wobei
die erste Teilzone (2601) und die zweite Teilzone (2603) aneinander grenzen, oder eine dritte Teilzone (2602) zwischen der ersten Teilzone (2601) und der zweiten Teilzone (2603) angeordnet ist, wobei in der dritten Teilzone (2602) die Haupteinschnitte (2612) in der Längsrichtung des proximalen passiven flexiblen Abschnitts (C) gleichmäßig voneinander beabstandet sind.

2. Endoskop nach Anspruch 1, wobei
die Haupteinschnitte (2611; 2613) parallel zueinander sind.

3. Endoskop nach Anspruch 1 oder 2, wobei
die sekundären Einschnitte (2621; 2623) jeweils benachbart zu einer Brücke (2631; 2633) zwischen Haupteinschnittteilen angeordnet sind, die auf einer Umfangslinie liegen,
insbesondere wobei
ein sekundärer Einschnitt (2621; 2623) benachbart zu jeder Brücke (2631; 2633) in der Längsrichtung des proximalen passiven flexiblen Abschnitts (C) auf einer Seite der Brücke (2631; 2633) angeordnet ist, oder
zwei sekundäre Einschnitte (2621; 2623) benachbart zu jeder Brücke (2631; 2633) in der Längsrichtung des proximalen passiven flexiblen Abschnitts (C) auf beiden Seiten der Brücke (2631; 2633) angeordnet sind.

4. Endoskop nach einem der Ansprüche 1 bis 3, wobei
die Haupteinschnitte (2611; 2613) breiter als die sekundären Einschnitte (2621; 2623) sind.

5. Endoskop nach einem der Ansprüche 1 bis 4, wobei
der gesamte Einführschlauch (2) einschließlich eines Verbindungsteils (D) des proximalen passiven flexiblen Abschnitts (C) zu einem Steuerkörper (3), des proximalen passiven flexiblen Abschnitts (C), eines Übergangsteils (B) zwischen dem proximalen passiven flexiblen Abschnitt (C) und dem Biegeabschnitt (A) und des Biegeabschnitts (A) aus einem einzelnen Rohrelement hergestellt ist.

6. Endoskop nach einem der Ansprüche 1 bis 5, wobei
der gesamte Einführschlauch (2) mittels Laser eingeschnitten ist.

7. Verfahren zum Herstellen eines Einführschlauchs (2) eines Endoskops aus einem Rohrelement,
wobei der Einführschlauch (2) einen proximalen passiven flexiblen Abschnitt (C) und einen distalen Biegeabschnitt (A) aufweist,
wobei Einschnitte (2611, 2621; 2613, 2623) in dem proximalen passiven flexiblen Abschnitt (C) vorgesehen werden, um ein Biegen des proximalen passiven flexiblen Abschnitts (C) zu ermöglichen,
wobei:
die Einschnitte (2611, 2621; 2613, 2623) in dem proximalen passiven flexiblen Abschnitt (C) so ausgebildet werden, dass benachbarte Einschnitte (2611, 2621; 2613, 2623) ungleichmäßig beabstandet sind,
sekundäre Einschnitte (2621; 2623) in dem proximalen passiven flexiblen Abschnitt (C) benachbart zu Haupteinschnitten (2611; 2613) vorgesehen werden, wobei die sekundären Einschnitte (2621; 2623) in einer Längsrichtung des proximalen passiven flexiblen Abschnitts (C) näher zu den benachbarten Haupteinschnitten (2611; 2613) auf einer Seite der sekundären Einschnitte (2621; 2623) als zu den benachbarten Haupteinschnitten (2611; 2613) auf der anderen Seite der sekundären Einschnitte (2621; 2623) angeordnet sind, und
die Haupteinschnitte (2611; 2613) entlang des Umfangs des proximalen passiven flexiblen Abschnitts (C) in einer unterbrochenen Weise eingeschnitten werden, so dass nicht eingeschnittene Brücken (2631; 2633) zwischen Haupteinschnittteilen verbleiben, die auf einer Umfangslinie liegen,
wobei die Haupteinschnitte (2611; 2613) in dem proximalen passiven flexiblen Abschnitt (C) vorgesehen werden, wobei zumindest innerhalb einer Teilzone die Haupteinschnitte (2611; 2613) in der Längsrichtung des proximalen passiven flexiblen Abschnitts (C) ungleichmäßig voneinander beabstandet sind,
**dadurch gekennzeichnet, dass**
zumindest innerhalb einer ersten Teilzone (2601) in dem proximalen passiven flexiblen Abschnitt (C) die Haupteinschnitte (2611; 2613) in der Längsrichtung des proximalen passiven flexiblen Abschnitts (C) mit kontinuierlich zunehmendem Abstand voneinander beabstandet sind, und
zumindest innerhalb einer zweiten Teilzone (2603) in dem proximalen passiven flexiblen Abschnitt (C) die Haupteinschnitte (2611; 2613) in der Längsrichtung des proximalen passiven flexiblen Abschnitts (C) mit kontinuierlich abnehmendem Abstand (H) voneinander beabstandet sind,
wobei
die erste Teilzone (2601) und die zweite Teilzone (2603) aneinander grenzen, oder
eine dritte Teilzone (2602) zwischen der ersten Teilzone (2601) und der zweiten Teilzone (2603) angeordnet ist, wobei in der dritten Teilzone (2602) die Haupteinschnitte (2611; 2613) in der Längsrichtung des proximalen passiven flexiblen Abschnitts (C) gleichmäßig voneinander beabstandet sind.

8. Verfahren nach Anspruch 7, wobei
die Haupteinschnitte (2611; 2613) parallel zueinander eingeschnitten werden.

9. Verfahren nach Anspruch 7 oder 8, wobei
die sekundären Einschnitte (2621; 2623) jeweils benachbart zu einer Brücke (2631; 2633) zwischen Haupteinschnittteilen eingeschnitten werden, die auf einer Umfangslinie liegen,
insbesondere wobei
ein sekundärer Einschnitt (2621; 2623) benachbart zu jeder Brücke (2631; 2633) in der Längsrichtung des proximalen passiven flexiblen Abschnitts (C) auf einer Seite der Brücke (2631; 2633) eingeschnitten wird, oder
zwei sekundäre Einschnitte (2621; 2623) benachbart zu jeder Brücke (2631; 2633) in der Längsrichtung des proximalen passiven flexiblen Abschnitts (C) auf beiden Seiten der Brücke (2631; 2633) eingeschnitten werden.

10. Verfahren nach einem der Ansprüche 7 bis 9, wobei
die Haupteinschnitte (2611; 2613) breiter als die sekundären Einschnitte (2621; 2623) eingeschnitten werden.

11. Verfahren nach einem der Ansprüche 7 bis 10, wobei
der gesamte Einführschlauch (2) einschließlich eines Verbindungsteils (D) des proximalen passiven flexiblen Abschnitts (C) zu einem Steuerkörper (3), des proximalen passiven flexiblen Abschnitts (C), eines Übergangsteils (B) zwischen dem proximalen passiven flexiblen Abschnitt (C) und dem Biegeabschnitt (A) und des Biegeabschnitts (A) aus einem einzelnen Rohrelement hergestellt wird, und/oder
der gesamte Einführschlauch (2) mittels Laser eingeschnitten wird.

## Revendications

1. Endoscope avec un tube d'insertion (2),
dans lequel le tube d'insertion (2) comprend une section flexible passive proximale (C) et une section de flexion distale (A),
des entailles (2611, 2621; 2613, 2623) sont prévues dans la section flexible passive proximale (C) pour permettre la flexion de la section flexible passive proximale (C),
des entailles adjacentes (2611, 2621 ; 2613, 2623) dans la section flexible passive proximale (C) sont espacées de manière inégale,
la section flexible passive proximale (C) comprend des entailles secondaires (2621 ; 2623) adjacentes aux entailles principales (2611 ; 2613), dans lequel les entailles secondaires (2621 ; 2623) sont agencées plus près (N) dans une direction longitudinale de la section flexible passive proximale (C) des entailles principales adjacentes (2611 ; 2613) sur un côté des entailles secondaires (2621 ; 2623) que des entailles principales adjacentes (2611 ; 2613) sur l'autre côté des entailles secondaires (2621 ; 2623), et
les entailles principales (2611 ; 2613) s'étendent le long de la circonférence de la section flexible passive proximale (C) d'une manière interrompue de sorte que des ponts non découpés (2631 ; 2633) restent entre des parties de entaille principale (2611 ; 2613) se trouvant sur une ligne circonférentielle,
dans lequel la section flexible passive proximale (C) comprend les entailles principales (2611 ; 2613), dans lequel au moins à l'intérieur d'une sous-zone dans la section flexible passive proximale (C), les entailles principales (2611 ; 2613) sont espacées de manière inégale les unes des autres dans la direction longitudinale de la section flexible passive proximale (C),
**caractérisé en ce que**
au moins à l'intérieur d'une première sous-zone (2601) dans la section flexible passive proximale (C), les entailles principales (2611 ; 2613) sont espacées les unes des autres dans la direction longitudinale de la section flexible passive proximale (C) avec un espacement augmentant en continu, et
au moins à l'intérieur d'une seconde sous-zone (2603) dans la section flexible passive proximale (C), les entailles principales (2611 ; 2613) sont espacées les unes des autres dans la direction longitudinale de la section flexible passive proximale (C) avec un espacement diminuant en continu,
dans lequel
la première sous-zone (2601) et la seconde sous-zone (2603) sont adjacentes l'une à l'autre, ou
une troisième sous-zone (2602) est agencée entre la première sous-zone (2601) et la seconde sous-zone (2603), dans lequel les entailles principales (2612) sont espacées de manière égale les unes des autres dans la direction longitudinale de la section flexible passive proximale (C) dans la troisième sous-zone (2602).

2. Endoscope selon la revendication 1, dans lequel
les entailles principales (2611 ; 2613) sont parallèles les unes aux autres.

3. Endoscope selon la revendication 1 ou 2, dans lequel
les entailles secondaires (2621; 2623) sont chacune agencées adjacentes à un pont (2631; 2633) entre des parties de entaille principale se trouvant sur une ligne circonférentielle, en particulier dans lequel
une entaille secondaire (2621 ; 2623) est agencée adjacente à chaque pont (2631 ; 2633) dans la direction longitudinale de la section flexible passive proximale (C) sur un côté du pont (2631 ; 2633), ou
deux entailles secondaires (2621 ; 2623) sont agencées adjacentes à chaque pont (2631 ; 2633) dans la direction longitudinale de la section flexible passive proximale (C) sur les deux côtés du pont (2631 ; 2633).

4. Endoscope selon l'une quelconque des revendications 1 à 3, dans lequel
les entailles principales (2611 ; 2613) sont plus larges que les entailles secondaires (2621; 2623).

5. Endoscope selon l'une quelconque des revendications 1 à 4, dans lequel
la totalité du tube d'insertion (2) comprenant une partie de raccordement (D) de la section flexible passive proximale (C) à un corps de commande (3), la section flexible passive proximale (C), une partie de transition (B) entre la section flexible passive proximale (C) et la section de flexion (A), et la section de flexion (A) est constituée d'un élément de tuyau unique.

6. Endoscope selon l'une quelconque des revendications 1 à 5, dans lequel
la totalité du tube d'insertion (2) est découpée par laser.

7. Procédé de fabrication d'un tube d'insertion (2) d'un endoscope à partir d'un élément de tuyau,
dans lequel le tube d'insertion (2) comprend une section flexible passive proximale (C) et une section de flexion distale (A),
dans lequel des entailles (2611, 2621 ; 2613, 2623) sont prévues dans la section flexible passive proximale (C) pour permettre la flexion de la section flexible passive proximale (C),
dans lequel :
les entailles (2611, 2621 ; 2613, 2623) dans la section flexible passive proximale (C) sont formées de sorte que des entailles adjacentes (2611, 2621 ; 2613, 2623) sont espacées de manière inégale,
des entailles secondaires (2621 ; 2623) sont prévues dans la section flexible passive proximale (C) adjacentes aux entailles principales (2611 ; 2613), dans lequel les entailles secondaires (2621 ; 2623) sont agencées plus près dans une direction longitudinale de la section flexible passive proximale (C) des entailles principales adjacentes (2611 ; 2613) sur un côté des entailles secondaires (2621 ; 2623) que des entailles principales adjacentes (2611 ; 2613) sur l'autre côté des entailles secondaires (2621 ; 2623), et
les entailles principales (2611 ; 2613) sont découpées le long de la circonférence de la section flexible passive proximale (C) d'une manière interrompue de sorte que des ponts non découpés (2631 ; 2633) restent entre des parties de entaille principale se trouvant sur une ligne circonférentielle,
dans lequel les entailles principales (2611 ; 2613) sont prévues dans la section flexible passive proximale (C), dans lequel au moins à l'intérieur d'une sous-zone, les entailles principales (2611 ; 2613) sont espacées de manière inégale les unes des autres dans la direction longitudinale de la section flexible passive proximale (C),
**caractérisé en ce que**
au moins à l'intérieur d'une première sous-zone (2601) dans la section flexible passive proximale (C), les entailles principales (2611 ; 2613) sont espacées les unes des autres dans la direction longitudinale de la section flexible passive proximale (C) avec un espacement augmentant en continu, et
au moins à l'intérieur d'une seconde sous-zone (2603) dans la section flexible passive proximale (C), les entailles principales (2611 ; 2613) sont espacées les unes des autres dans la direction longitudinale de la section flexible passive proximale (C) avec un espacement diminuant en continu (H),
dans lequel
la première sous-zone (2601) et la seconde sous-zone (2603) sont adjacentes l'une à l'autre, ou
une troisième sous-zone (2602) est agencée entre la première sous-zone (2601) et la seconde sous-zone (2603), dans lequel les entailles principales (2611 ; 2613) sont espacées de manière égale les unes des autres dans la direction longitudinale de la section flexible passive proximale (C) dans la troisième sous-zone (2602).

8. Procédé selon la revendication 7, dans lequel
les entailles principales (2611 ; 2613) sont découpées parallèlement les unes aux autres.

9. Procédé selon la revendication 7 ou 8, dans lequel
les entailles secondaires (2621 ; 2623) sont chacune découpées adjacentes à un pont (2631 ; 2633) entre des parties de entaille principale se trouvant sur une ligne circonférentielle,
en particulier dans lequel
une entaille secondaire (2621 ; 2623) est découpée adjacente à chaque pont (2631 ; 2633) dans la direction longitudinale de la section flexible passive proximale (C) sur un côté du pont (2631 ; 2633), ou
deux entailles secondaires (2621; 2623) sont découpées adjacentes à chaque pont (2631; 2633) dans la direction longitudinale de la section flexible passive proximale (C) sur les deux côtés du pont (2631 ; 2633).

10. Procédé selon l'une quelconque des revendications 7 à 9, dans lequel
les entailles principales (2611 ; 2613) sont découpées plus larges que les entailles secondaires (2621 ; 2623).

11. Procédé selon l'une quelconque des revendications 7 à 10, dans lequel
la totalité du tube d'insertion (2) comprenant une partie de raccordement (D) de la section flexible passive proximale (C) à un corps de commande (3), la section flexible passive proximale (C), une partie de transition (B) entre la section flexible passive proximale (C) et la section de flexion (A), et la section de flexion (A) est constituée d'un élément de tuyau unique, et/ou
la totalité du tube d'insertion (2) est découpée par laser.
